(19) Europäisches Patentamt
European Patent Office
Office européen des brevets

(11) EP 4 442 247 A1

(12) EUROPEAN PATENT APPLICATION
published in accordance with Art. 153(4) EPC

(43) Date of publication:
**09.10.2024 Bulletin 2024/41**

(21) Application number: **22901244.8**

(22) Date of filing: **28.11.2022**

(51) International Patent Classification (IPC):
***A61K 8/89*** (2006.01) ***A61K 8/891*** (2006.01)
***A61K 8/893*** (2006.01) ***A61Q 3/02*** (2006.01)
***A61Q 5/10*** (2006.01)

(52) Cooperative Patent Classification (CPC):
**A61K 8/58; A61K 8/89; A61K 8/891; A61K 8/893; A61Q 3/02; A61Q 5/00; A61Q 5/10**

(86) International application number:
**PCT/JP2022/043784**

(87) International publication number:
**WO 2023/100812 (08.06.2023 Gazette 2023/23)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.11.2021 JP 2021194587**

(71) Applicant: **Kao Corporation**
**Tokyo 103-8210 (JP)**

(72) Inventors:
- **MOGAKI, Rina**
  **Tokyo 131-8501 (JP)**
- **MAEKAWA, Tomoka**
  **Tokyo 131-8501 (JP)**

(74) Representative: **Hoffmann Eitle**
**Patent- und Rechtsanwälte PartmbB**
**Arabellastraße 30**
**81925 München (DE)**

# (54) METHOD FOR TREATING KERATIN SUBSTANCE OR FIBER FOR HEAD ORNAMENTAL PRODUCT

(57) Provided is a method for treating an artificially colored or decolored keratin substance or fiber for a head decoration product with a composition, the method including a step of applying the composition to the keratin substance or the fiber for a head decoration product, wherein the composition comprises the following component (A) and component (B): (A) a silicone film-forming agent; (B) an organopolysiloxane having a degree of polymerization of 100 or more, and in which a ratio of a content mass of the component (A) to a total content mass of the component (A) and the component (B) [(A)/{(A) + (B)}] is 10% or more.

## Description

Field of the Invention

The present invention relates to a method for treating a keratin substance or a fiber for a head decoration product.

Background of the Invention

Keratin substances are sometimes colored or decolored by artificial means for the purpose of improving fashionability, such as dyeing hair with a hair dye, bleaching hair with a bleaching agent, and dyeing nails with a manicure. However, the coloring or decoloring effect may be lost by friction due to daily living activities, discoloration with the passage of time, or one time washing, and methods for maintaining the coloring or decoloring applied to keratin substances or fibers for head decoration products such as wigs have been studied.

For example, JP 2013-541585 A (PTL 1) discloses a method for improving color retention in artificially colored hair, which includes applying to artificially colored hair a composition containing a hydrophobic particle substance having a predetermined particle diameter, a silicone-based hydrophobic film-forming agent, and a medium containing a volatile hydrocarbon or a silicone solution, in which a content of a non-volatile, water-soluble or water-dispersible organic component is less than a predetermined value.

Summary of the Invention

The present invention relates to the following.

[1] A method for treating an artificially colored or decolored keratin substance or fiber for a head decoration product with a composition, the method including a step of applying the composition to the keratin substance or the fiber for a head decoration product, wherein the composition comprises the following component (A) and component (B):

(A) a silicone film-forming agent;
(B) an organopolysiloxane having a degree of polymerization of 100 or more, and

wherein a proportion of a content mass of the component (A) to a total content mass of the component (A) and the component (B) [(A)/ f (A) + (B)}] is 10% or more.

[2] A kit for treating a keratin substance or a fiber for a head decoration product, including a first agent comprising one or more selected from the group consisting of a colorant and a decolorant, and a second agent comprising the following component (A) and component (B), wherein a proportion of a content mass of the component (A) to a total content mass of the component (A) and the component (B) [(A)/ f (A) + (B)}] is 10% or more:

(A) a silicone film-forming agent;
(B) an organopolysiloxane having a degree of polymerization of 100 or more.

Detailed Description of the Invention

In the disclosed technique of PTL 1, it is described that the composition is applied to color-treated hair (wet hair or dry hair) after each shampoo. However, in this method, it is necessary to apply the composition to the hair every time shampooing is performed, and there is room for improvement in the durability of the coloring effect.

In addition, from the viewpoint of further improving the color durability by reducing the number of times of washing of the colored keratin substance, it is preferable that the keratin substance after the treatment can be provided with resistance to adhesion of dirt (antifouling property). However, the imparting of antifouling property to the keratin substance has not been examined in PTL 1.

The present invention relates to a method for treating an artificially colored or decolored keratin substance or fiber for a head decoration product, in which the color durability of the keratin substance or the fiber for a head decoration product can be improved even by a single treatment, and an effect of preventing adhesion of dirt can be imparted.

The present inventors have found that the above-described problems can be solved by treating an artificially colored or decolored keratin substance or fiber for a head decoration product with a composition containing a silicone film-forming agent and organopolysiloxane having a degree of polymerization of 100 or more at a predetermined mass ratio, thereby completing the present invention.

According to the present invention, it is possible to improve the color durability of a keratin substance or a fiber for a head decoration product which has been artificially colored or decolored even by one time treatment, and it is also

possible to impart an effect of preventing adhesion of dirt.

[Definitions]

"Polymer" as used herein means a compound corresponding to a repetition of one or plural units (these units are derived from a compound known as a monomer). This or these units are repeated at least two times preferably at least three times.

"Keratin substance" as used herein includes, for example, the skin, eyebrows, eyelashes, hair, and nails of an animal such as a human being, as well as artificially produced keratin substances (excluding fibers for a head decoration product).

"Hair" as used herein means human head hair, but also includes hair that has been cut from a human head.

"Head decoration product" as used herein means, for example, a hair wig, a wig, weaving, hair extension, a blade hair, a hair accessory, and a doll hair.

"Fiber for a head decoration product" as used herein means a fiber used in the head decoration product.

"Hydrophobic" as used herein means that a solubility in water of a substance is less than 1% by mass at 25°C.

"Film formation" as used herein means that, when applied to a substrate, a solid film is left thereon.

"Volatile" as used herein means a substance having a boiling point of 260°C or lower under normal pressure.

[Method for Treating Keratin Substance or Fiber for Head Decoration Product]

The method for treating a keratin substance or a fiber for a head decoration product of the present invention (hereinafter also referred to as the "present method") is a method for treating an artificially colored or decolored keratin substance or fiber for a head decoration product with a composition, and the method includes a step of applying the composition to the keratin substance or the fiber for a head decoration product, in which the composition contains the following component (A) and component (B): (A) a silicone film-forming agent; (B) an organopolysiloxane having a degree of polymerization of 100 or more, and in which a proportion of a content mass of the component (A) to a total content mass of the component (A) and the component (B) [(A)/ f (A) + (B)}] is 10% or more.

According to the above-mentioned constitution of the present invention, it is possible to improve the color durability of a keratin substance or a fiber for a head decoration product which has been artificially colored or decolored even by one time treatment, and it is also possible to impart an effect of preventing adhesion of dirt. That is, the method of the present invention relates to a method for protecting the color of an artificially colored or decolored keratin substance or fiber for a head decoration product, and a method for imparting an effect of preventing adhesion of dirt to an artificially colored or decolored keratin substance or fiber for a head decoration product.

The term "one time treatment" as used herein means that the number of applications of the composition is one. However, in the method of the present invention, the artificially colored or decolored keratin substance or fiber for a head decoration product is not limited to being treated only once, and the treatment may be performed a plurality of times.

The reason why the present invention exhibits the above-mentioned effects is not clear, but is presumed as follows.

The composition used in the treatment method of the present invention can form a hydrophobic film on the surface of an artificially colored or decolored keratin substance or fiber for a head decoration product by containing the component (A) and the component (B). The component (A) is a hydrophobic film-forming agent, and since the strength and durability of the film can be maintained, it is considered that the color change of the keratin substance or the fiber for a head decoration product due to friction or washing can be suppressed. On the other hand, it is considered that a large amount of the component (B) is present on the film surface side (air side) in the film to be formed, and it is considered that by having a degree of polymerization of a predetermined value or more, the component (B) can perform a surface protective function for a keratin substance or a fiber for a head decoration product and can impart an effect of preventing adhesion of dirt. Since the component (A) and the component (B) have compatibility, it is considered that when the component (A) and the component (B) are applied to the surface of an artificially colored or decolored keratin substance or fiber for a head decoration product due to a synergistic effect thereof, the color durability can be improved and an effect of preventing adhesion of dirt can also be imparted.

In addition, it is considered that in a case where the proportion of a content mass of the component (A) to a total content mass of the component (A) and the component (B) [(A)/ f (A) + (B)}] is 10% or more, a film having higher durability against washing can be formed. Therefore, it is considered that the color durability and the effect of preventing adhesion of dirt of the artificially colored or decolored keratin substance or fiber for a head decoration product are obtained even by one time treatment.

It should be noted that the action mechanism of the present invention is not limited to the above.

<Artificially Colored or Decolored Keratin Substance or Fiber for Head Decoration Product>

The keratin substance to which the method of the present invention is applied preferably includes skin, eyebrows, eyelashes, hair, and nails. From the viewpoint of the effect of improving color durability, among these, skin, eyebrows, eyelashes, or hair is more preferable, and hair is still more preferable.

The fiber for a head decoration product to which the method of the present invention is applied may be either a naturally derived fiber or a synthetic fiber, and a naturally derived fiber is preferable. The naturally derived fiber refers to a fiber collected from a natural animal or plant (excluding human hair) or a fiber artificially produced using a protein or a polysaccharide as a raw material. Of these, fibers artificially produced from animal hair or proteins or polysaccharides such as proteins derived from keratin, collagen, casein, soybean, peanut, corn, and silk are preferable, regenerated protein fibers from keratin, collagen, casein, soybean protein, peanut protein, corn protein, and silk protein (for example, silk fibroin) are more preferable, regenerated protein fibers such as regenerated collagen fibers from collagen and regenerated silk fibers from silk fibroin are more preferable, and regenerated collagen fibers are still more preferable.

The regenerated collagen fibers can be produced by a known technique. The composition of the regenerated collagen fibers need not be 100% collagen, and may contain natural polymers, synthetic polymers, and additives for quality improvement. Furthermore, the regenerated collagen fibers may be post-treated.

The regenerated collagen fibers are preferably in the form of filaments. The filament is generally taken out from a bobbin-wound state or a packed state. It is also possible to directly utilize the filament coming out from the drying step in the production process of the regenerated collagen fiber.

Examples of the synthetic fiber include a fiber containing a synthetic resin as a main component. From the viewpoint of ease of production of the synthetic fiber and from the viewpoint of obtaining a texture close to that of hair, the synthetic resin is preferably a thermoplastic resin, and more preferably one or more selected from the group consisting of a polyester resin, a polyamide resin, a polyimide resin, a polyamide-imide resin, a vinyl chloride resin, a polycarbonate resin, a polyphenylene sulfide resin, and a modacrylic resin (a copolymer of acrylonitrile and vinyl chloride). The term "main component" as used herein means a component having a content in the synthetic fiber of preferably 50% by mass or more, more preferably 60% by mass or more, still more preferably 70% by mass or more, even more preferably 80% by mass or more, and even more preferably 90% by mass or more, and 100% by mass or less.

The synthetic fiber may further contain various components such as a flame retardant, a flame retardant aid, a light or heat stabilizer, a fluorescent agent, an antioxidant, an anti-static agent, and an ultraviolet absorber in addition to the synthetic resin within a range not inhibiting the effect of the present invention.

The "artificially colored keratin substance or fiber for a head decoration product" means a keratin substance or a fiber for a head decoration product which has been subjected to a coloring treatment using a colorant such as a pigment or a dye. The colorant is preferably one or more selected from the group consisting of pigments, direct dyes, and oxidation dyes from the viewpoint of improving color durability, more preferably one or more selected from the group consisting of pigments and direct dyes, and still more preferably pigments from the viewpoint of improving color durability, ease of treatment during coloring treatment, and less damage due to coloring treatment.

The pigment may be any pigment generally used in makeup cosmetic materials and hair dye compositions, and examples thereof include a white inorganic pigment such as titanium oxide, zinc oxide, cerium oxide and barium sulfate; a colored inorganic pigment such as yellow iron oxide, black iron oxide, red iron oxide, carbon black, chromium oxide, chromium hydroxide, Prussian blue and ultramarine blue; a luster powder such as titanium oxide-coated mica, titanium oxide-coated bismuth oxychloride, iron oxide-coated mica titanium, iron oxide mica, Prussian blue-processed mica titanium, carmine-processed mica titanium, bismuth oxychloride, and fish scale guanine; an organic pigment such as Red No. 201, Red No. 202, Red No. 205, Red No. 226, Red No. 228, Orange No. 203, Orange No. 204, Blue No. 404, and Yellow No. 401; a lake pigment such as a zirconium, barium or aluminum lake of Red No. 3, Red No. 104, Red No. 106, Orange No. 205, Yellow No. 4, Yellow No. 5, Green No. 3 or Blue No. 1; and a composite pigment such as fine particle titanium oxide-coated mica titanium, fine particle zinc oxide-coated mica titanium, barium sulfate-coated mica titanium, titanium oxide-containing silicon dioxide and zinc oxide-containing silicon dioxide. One or more of these can be used.

In addition, pigments whose surfaces are treated with various surface treatment agents may be used. The surface treatment is not specifically limited. Various surface treatments can be applied to the pigments, and examples thereof include fluorine compound treatment, silicone treatment, silicone resin treatment, pendant treatment, silane coupling agent treatment, titanium coupling agent treatment, oil treatment, metal soap treatment, N-acylated lysine treatment, polyethylene glycol treatment, PVA treatment, polyacrylic acid treatment, hyaluronic acid treatment, alginic acid treatment, inorganic compound treatment, plasma treatment and mechanochemical treatment.

Examples of the direct dye include an acid dye, a nitro dye, a disperse dye, a basic dye, and a direct dye described in JP 2003-342139 A.

Examples of the acidic dye include Blue No. 1, Violet No. 401, Black No. 401, Orange No. 205, Red No. 227, Red No. 106, Yellow No. 203, and Acid Orange 3. Examples of the nitro dye include 2-nitroparaphenylene diamine, 2-amino-6-chloro-4-nitrophenol, 3-nitro-p-hydroxyethylaminophenol, 4-nitroorthophenylene diamine, 4-amino-3-nitrophenol, 4-hydroxypropylamino-3-nitrophenol, HC Blue No. 2, HC Orange No. 1, HC Red No. 1, HC Yellow No. 2, HC Yellow No. 4, HC Yellow No. 5, HC Red No. 3, and N,N-bis-(2-hydroxyethyl)-2-nitroparaphenylene diamine. Examples of the disperse dye include Disperse Violet 1, Disperse Blue 1, and Disperse Black 9. Examples of the basic dye include Basic Blue 99, Basic Brown 16, Basic Brown 17, Basic Red 76, Basic Red 51, Basic Yellow 57, Basic Yellow 87, and Basic Orange 31. One or more of these direct dyes can be used.

Examples of the oxidation dye include dyes composed of a precursor and a coupler.

Examples of the precursor in the oxidation dye include one or more selected from the group consisting of paraphenylenediamine, toluene-2,5-diamine, 2-chloro-paraphenylenediamine, N-methoxyethyl paraphenylenediamine, N,N-bis(2-hydroxyethyl)paraphenylenediamine, 2-(2-hydroxyethyl)paraphenylenediamine, 2,6-dimethylparaphenylenediamine, 4,4'-diaminodiphenylamine, 1,3-bis(N-(2-hydroxyethyl)-N-(4-aminophenyl)amino)-2-propanol, PEG-3,3,2'-paraphenylenediamine, para-aminophenol, para-methylaminophenol, 3-methyl-4-aminophenol [= 4-aminometacresol], 2-aminomethyl-4-aminophenol, 2-(2-hydroxyethylaminomethyl)-4-aminophenol, ortho-aminophenol, 2-amino-5-methylphenol, 2-amino-6-methylphenol, 2-amino-5-acetamidophenol, 3,4-diaminobenzoic acid, 5-aminosalicylic acid, 2,4,5,6-tetraaminopyrimidine, 2,5,6-triamino-4-hydroxypyrimidine, 4,5-diamino-1-(4'-chlorobenzyl)pyrazole, 1-hydroxyethyl-4,5-diaminopyrazole, and salts thereof.

Examples of the coupler in the oxidation dye include one or more selected from the group consisting of metaphenylenediamine, 2,4-diaminophenoxyethanol, 2-amino-4-(2-hydroxyethylamino)anisole [= 2-amino-4-(β-hydroxyethyl)aminoanisole], 2,4-diamino-5-methylphenetole, 2,4-diamino-5-(2-hydroxyethoxy)toluene, 2,4-dimethoxy-1,3-diaminobenzene, 2,6-bis(2-hydroxyethylamino)toluene, 2,4-diamino-5-fluorotoluene, 1,3-bis(2,4-diaminophenoxy)propane, metaaminophenol, 2-methyl-5-aminophenol [= 5-aminoorthocresol], 2-methyl-5-(2-hydroxyethylamino)phenol, 2,4-dichloro-3-aminophenol, 2-chloro-3-amino-6-methylphenol, 2-methyl-4-chloro-5-aminophenol, N-cyclopentyl-metaaminophenol, 2-methyl-4-methoxy-5-(2-hydroxyethylamino)phenol, 2-methyl-4-fluoro-5-aminophenol, resorcin, 2-methylresorcin, 4-chlororesorcin, 1-naphthol, 1,5-dihydroxynaphthalene, 1,7-dihydroxynaphthalene, 2,7-dihydroxynaphthalene, 2-isopropyl-5-methylphenol, 4-hydroxyindole, 5-hydroxyindole, 6-hydroxyindole, 7-hydroxyindole, 6-hydroxybenzomorpholine, 3,4-methylenedioxyphenol, 2-bromo-4,5-methylenedioxyphenol, 3,4-methylenedioxyaniline, 1-(2-hydroxyethyl)amino-3,4-methylenedioxybenzene, 2,6-dihydroxy-3,4-dimethylpyridine, 2,6-dimethoxy-3,5-diaminopyridine, 2,3-diamino-6-methoxypyridine, 2-methylamino-3-amino-6-methoxypyridine, 2-amino-3 -hydroxypyridine, 2,6-diaminopyridine, and salts thereof.

Examples of the "artificially decolored keratin substance or fiber for a head decoration product" include a keratin substance or fiber for a head decoration product artificially bleached with a bleaching agent.

The object to which the method of the present invention is applied is preferably artificially colored or decolored skin, eyebrows, eyelashes, hair, nails, or fibers for head decoration products, more preferably artificially colored or decolored hair or fibers for head decoration products, still more preferably artificially colored or decolored hair, and even more preferably artificially colored hair, and examples thereof include hair dyed with a hair dye. The hair dye is a hair dye containing preferably a colorant, more preferably one or more selected from the group consisting of a pigment, a direct dye and an oxidation dye, and still more preferably one or more selected from the group consisting of a pigment and a direct dye, from the viewpoint of improving color durability, and is even more preferably a hair dye containing a pigment, from the viewpoint of improving color durability, ease of treatment during coloring treatment, and less damage due to coloring treatment.

The hair to be colored may be any of white hair, bleached hair, and colored hair.

<Composition>

The composition used in the present invention contains the following component (A) and component (B): (A) a silicone film-forming agent; (B) an organopolysiloxane having a degree of polymerization of 100 or more, in which a proportion of a content mass of the component (A) to a total content mass of the component (A) and the component (B) [(A)/ f (A) + (B)}] is 10% or more.

<Component (A): Silicone Film-Forming Agent>

The composition contains a silicone film-forming agent as the component (A). By containing the component (A), when the composition is applied to a keratin substance or a fiber for a head decoration product, it is possible to form a hydrophobic film which has high durability to friction and washing, and can improve the color durability of the artificially colored or decolored keratin substance or fiber for a head decoration product.

As the component (A), a silicone film-forming agent usually used in cosmetics can be used, but a silicone film-forming agent which is solid at 25°C is preferred.

The component (A) is preferably one or more selected from the group consisting of the following component

(A1) and component (A2) from the viewpoint of improving film formability and color durability.

(A1) A silicone resin represented by an average formula, $(R^1)_mSiO_{(4-m)/2}$ (in which $R^1$ represents a hydrocarbon group having 1 or more and 12 or less carbon atoms and optionally substituted with fluorine, or a hydroxy group, plural $R^1$'s can be the same as or different from each other, and m is an average number, representing a number of more than 0 and less than 3), which contains one or more units selected from the group consisting of a T unit represented by $R^1SiO_{3/2}$ and a Q unit represented by $SiO_{4/2}$.
(A2) A silicone polymer containing a polysiloxane moiety and a moiety formed of a non-silicone organic chain.

Preferably, the component (A1) includes those containing one or more selected from the group consisting of the following component (A1-1) and component (A1-2).

(A1-1) A silicone resin represented by the above-mentioned average formula, containing a T unit represented by $R^1SiO_{3/2}$ and substantially not containing a Q unit represented by $SiO_{4/2}$.
(A1-2) A silicone resin represented by the above-mentioned average formula, and containing a Q unit represented by $SiO_{4/2}$ and an M unit represented by $(R^1)_3SiO_{1/2}$.

Preferably, the component (A2) includes those containing one or more selected from the group consisting of the following component (A2-1) to (A2-4).

(A2-1) An acryl silicone polymer.
(A2-2) A silicone-modified alicyclic structure-containing polymer.
(A2-3) A silicone-modified pullulan.
(A2-4) A polyurea/urethane silicone.

(Component (A1))

The component (A1) is a silicone resin represented by an average formula, $(R^1)_mSiO_{(4-m)/2}$ (in which $R^1$ represents a hydrocarbon group having 1 or more and 12 or less carbon atoms and optionally substituted with fluorine, or a hydroxy group, plural $R^1$'s can be the same as or different from each other, and m is an average number, representing a number of more than 0 and less than 3), which contains one or more units selected from the group consisting of a T unit represented by $R^1SiO_{3/2}$ and a Q unit represented by $SiO_{4/2}$.

The component (A1) is represented by the above-mentioned average formula and contains one or more units selected from the group consisting of the above-mentioned T unit and Q unit, and therefore has a crosslinked structure in the molecule. Having the structure, the silicone resin is considered to be able to form a film having higher durability. The component (A1) does not contain a polyorganosiloxane cured product powder which is infusible and does not have a softening point and which is generally insoluble in an organic solvent.

In the average formula, $R^1$ represents a hydrocarbon group having 1 or more and 12 or less carbon atoms and optionally substituted with fluorine, or a hydroxy group. The number of carbon atoms of the hydrocarbon group is 1 or more, and is preferably 9 or less, more preferably 6 or less, and still more preferably 4 or less, from the viewpoint of improving film formability and durability.

The hydrocarbon group may be any of an aliphatic group or an aromatic group, and examples thereof include an alkyl group, an alkenyl group, an aryl group, and an aralkyl group. The alkyl group and the alkenyl group may be linear or branched.

Among the above, from the viewpoint of availability and stability, the hydrocarbon group is preferably an alkyl group, an aryl group, or an aralkyl group.

The alkyl group includes a methyl group, an ethyl group, a n-propyl group, an isopropyl group, various butyl groups, various pentyl groups, various hexyl groups, various heptyl groups, various octyl groups, various nonyl group, various decyl groups, various undecyl groups, and various dodecyl groups. The word "various" means a linear or branched hydrocarbon group, and for example, "various butyl groups" include "a n-butyl group, a sec-butyl group, an isobutyl group, and a tert-butyl group".

The aryl group includes a phenyl group, a toluyl group, a dimethylphenyl group, and a naphthyl group, and is preferably a phenyl group.

The aralkyl group includes a benzyl group, a phenylethyl group, a phenylpropyl group, and a phenylbutyl group, and is preferably a phenylpropyl group.

In the case where $R^1$ is substituted with fluorine, at least one hydrogen atom of the hydrocarbon group may be substituted with a fluorine atom.

$R^1$ is, from the viewpoint of improving film formability and color durability, preferably an optionally fluorine-substituted, alkyl group having 1 or more and 12 or less carbon atoms, aryl group having 6 or more and 12 or less carbon atoms or aralkyl group having 7 or more and 12 or less carbon atoms, more preferably an optionally fluorine-substituted, alkyl group having 1 or more and 8 or less carbon atoms or phenyl group, even more preferably an optionally fluorine-substituted, alkyl group having 1 or more and 6 or less carbon atoms or phenyl group. The fluorine-substituted alkyl group is preferably a group represented by $CF_3$-R- in which R represents an alkylene group having 2 or more and 7 or less carbon atoms, preferably 2 or more and 5 or less carbon atoms.

$R^1$ is even more preferably a trifluoropropyl group, an alkyl group having 1 or more and 4 or less carbon atoms, or a phenyl group, even more preferably a trifluoropropyl group, a methyl group, an ethyl group, a n-propyl group, an isopropyl group or a n-butyl group, even more preferably a trifluoropropyl group, a methyl group or a n-propyl group, and even more preferably a methyl group.

The component (A1) may contain one or more units selected from the group consisting of a T unit represented by $R^1SiO_{3/2}$ and a Q unit represented by $SiO_{4/2}$ and, from the viewpoint of improving film formability and color durability, preferably further contains one or more units selected from the group consisting of an M unit represented by $(R^1)_3SiO_{1/2}$ and a D unit represented by $(R^1)_2SiO_{2/2}$. $R^1$ is the same as above.

From the viewpoint of improving film formability and color durability, the component (A1) is preferably one or more selected from the group consisting of a silicone resin (A1-1) represented by the above-mentioned average formula, containing a T unit represented by $R^1SiO_{3/2}$ and substantially not containing a Q unit represented by $SiO_{4/2}$, and a silicone resin (A1-2) represented by the above-mentioned average formula, and containing a Q unit represented by $SiO_{4/2}$ and an M unit represented by $(R^1)_3SiO_{1/2}$.

[Silicone Resin (A1-1)]

The silicone resin (A1-1) represented by the above-mentioned average formula, containing a T unit represented by $R^1SiO_{3/2}$ and substantially not containing a Q unit represented by $SiO_{4/2}$ (hereinafter also referred to as "component (A1-1)") is a silicone resin containing a T unit and optionally containing an M unit and a D unit, and is preferably a silicone resin represented by $[R^1SiO_{3/2}]_a[(R^1)_3SiO_{1/2}]_b$ in which a and b each are an average repeating unit number, and $a>0$ and $b\geq0$, and containing a T unit and optionally containing an M unit. The wording "substantially not containing XX" means the constituent ratio of XX in the silicone resin is less than 1 mol%.

$R^1$ is the same as above, and is preferably an alkyl group having 1 or more and 4 or less carbon atoms or a phenyl group, more preferably a methyl group, an ethyl group, a n-propyl group or an isopropyl group, and even more preferably a methyl group, a n-propyl group, or an isopropyl group.

The component (A1-1) includes polysilsesquioxanes such as polymethylsilsesquioxane, polypropylsilsesquioxane, polyphenylsilsesquioxane, polymethylphenylsilsesquioxane, and fluorine-modified alkyldimethylpolysilsesquioxanes, and among these, one or more can be used. Fluorine-modified alkyldimethylpolysilsesquioxanes include, as INCI nomenclature, (trifluoropropyldimethylsiloxy/trimethylsiloxy)silsesquioxane.

Among these, as the component (A1-1), one or more selected from the group consisting of polymethylsilsesquioxane and polypropylsilsesquioxane is preferable, and polymethylsilsesquioxane is more preferable, from the viewpoint of improving film formability and color durability.

Commercially available products of the component (A1-1) include SilForm Flexible Resin (polymethylsilsesquioxane), SilForm FR-5 (polydimethylsiloxane solution of (trifluoropropyldimethylsiloxy/trimethylsiloxy)silsesquioxane) (all manufactured by Momentive Performance Materials, Inc.), DOWSIL 680 ID Fluid (isododecane solution of 75% by mass polypropylsilsesquioxane) (manufactured by Dow Toray Co., Ltd.), SR-21 (polyphenylsilsesquioxane), SR-23 (polyphenylsilsesquioxane), SR-33 (polymethylphenylsilsesquioxane) (all manufactured by Konishi Chemical Ind. Co., Ltd.).

[Silicone Resin (A1-2)]

The silicone resin (A1-2) represented by the above-mentioned average formula, and containing a Q unit represented by $SiO_{4/2}$ and an M unit represented by $(R^1)_3SiO_{1/2}$ (hereinafter also referred to as "component (A1-2)") is a silicone resin substantially containing a Q unit and an M unit and optionally containing a D unit or a T unit, and is preferably a silicone resin represented by $[SiO_{4/2}]_c[(R^1)_3SiO_{1/2}]_d$ in which c and d each are an average repeating unit number and $c > 0$ and $d > 0$.

$R^1$ is the same as above, and is preferably an optionallyfluorine-substituted, alkyl group having 1 or more and 6 or less carbon atoms or phenyl group, more preferably a trifluoropropyl group, an alkyl group having 1 or more and 4 or less carbon atoms, or a phenyl group, even more preferably a trifluropropyl group, a methyl group, an ethyl group, a n-propyl group, or an isopropyl group, even more preferably a trifluoropropyl group or a methyl group, and even more preferably a methyl group.

The component (A1-2) includes trimethylsiloxysilicate, phenylpropyldimethylsiloxysilicate, fluorine-modified alkyldimethylsiloxysilicates, and crosspolymers produced by crosslinking these siloxysilicates with dimethiconol, and

one or more of these can be used. Fluorine-modified alkyldimethylsiloxysilicates include trifluoroalkyldimethyltrimethylsiloxysilicate, such as trifluoropropyldimethyltrimethylsiloxysilicate of, as INCI nomenclature, trifluoropropyldimethyl/trimethylsiloxysilicate. Crosspolymers produced by crosslinking siloxysilicates with dimethiconol include, as INCI nomenclature, (trimethylsiloxysilicate/dimethiconol) crosspolymer.

Above all, from the viewpoint of improving film formability and color durability, the component (A1-2) is preferably one or more selected from the group consisting of trimethylsiloxysilicate, trifluoropropyldimethyltrimethylsiloxysilicate and (trimethylsiloxysilicate/dimethiconol) crosspolymer, more preferably one or more selected from the group consisting of trimethylsiloxysilicate, and trifluoropropyldimethyltrimethylsiloxysilicate, and even more preferably trimethylsiloxysilicate.

Commercially available products of trimethylsiloxysilicate of the component (A1-2) include KF-7312J (50% by mass decamethylcyclopentasiloxane solution), KF-9021 (50% by mass decamethylcyclopentasiloxane solution), X-21-5249(50% by mass decamethylcyclopentasiloxane solution), X-21-5595 (60% by mass isododecane solution), and X-21-5616 (60% by mass isododecane solution) (all manufactured by Shin-Etsu Chemical Co., Ltd.), SS4267 (35% by mass dimethylpolysiloxane solution), SR1000, SS4230 (45% by mass cyclopentasiloxane solution), SS4267 (35% by mass dimethylpolysiloxane solution), and Silsoft 74 (75% by mass isododecane solution) (all manufactured by Momentive Performance Materials, Inc.), BY11-018 (30% by mass cyclopentasiloxane solution), and MQ-1600 Solid Resin (all manufactured by Dow Toray Co., Ltd.), and BELSIL TMS 803 (manufactured by Wacker Asahikasei Silicone Co., Ltd.).

Commercially available products of phenylpropyldimethylsiloxysilicate include SilShine 151 (manufactured by Momentive Performance Materials, Inc.).

Commercially available products of fluorine-modified alkyldimethylsiloxysilicates include, XS66-B8226 (50% by mass cyclopentasiloxane solution), XS66-C1191, and XS66-B8636 (50% by mass dimethicone solution) (all manufactured by Momentive Performance Materials, Inc.), as INCI nomenclature, (trifluoropropyldimethyl/trimethylsiloxysilicate).

Commercially available products of trimethylsiloxysilicate crosspolymer include DOWSIL FC-5001 CM Resin Gum (40% by mass cyclopentasiloxane solution of (trimethylsiloxysilicate/dimethiconol) crosspolymer), DOWSIL FC-5002 IDD Resin Gum (40% by mass isododecane solution of (trimethylsiloxysilicate/dimethiconol) crosspolymer), and DOWSIL FC-5004 DM (1.5 cSt) Silicone Resin Gum (40% by mass dimethicone solution of (trimethylsiloxysilicate/dimethiconol) crosspolymer) (all manufactured by Dow Toray Co., Ltd.).

In addition, commercially available products of the mixture of the silicone resin (A1-1) and the silicone resin (A1-2) include DOWSIL MQ-1640 Flake Resin (a mixture of trimethylsiloxysilicate and polypropylsilsesquioxane, manufactured by Dow Toray Co., Ltd.).

(Component (A2))

The component (A2) is a silicone polymer containing a polysiloxane moiety and a moiety formed of a non-silicone organic chain. The non- silicone organic monomer to constitute the moiety formed of a non-silicone organic chain is, from the viewpoint of availability on the market, preferably selectable from a radical-polymerizable ethylenically-unsaturated monomer, a polycondensation-polymerizable monomer (e.g., those to form polyamides, polyesters or polyurethanes), and a ring-cleavable monomer (e.g., oxazoline or caprolactone-type ones).

Preferably, the component (A2) includes those containing one or more selected from the group consisting of the following components (A2-1) to (A2-4), more preferably those containing the component (A2-1).

(A2-1) An acryl silicone polymer.
(A2-2) A silicone-modified alicyclic structure-containing polymer.
(A2-3) A silicone-modified pullulan.
(A2-4) A polyurea/urethane silicone.

[Acryl Silicone Polymer (A2-1)]

The acryl silicone polymer of the component (A2-1) includes an acrylic polymer having a carbosiloxane dendrimer structure in the side chain, an acryl-silicone graft copolymer, and a graft-type copolymer or alternate block-type copolymer where a structural unit of a polysiloxane group and a structural unit of a polymer of an unsaturated monomer via a sulfide bond.

The acrylic polymer having a carbosiloxane dendrimer structure in the side chain includes a silicone dendrimer-acryl copolymer, and for example, can be produced according to the production method described in JP H11-1530 A and JP 2000-63225 A.

The acrylic polymer having a carbosiloxane dendrimer structure in the side chain is preferably, as INCI nomenclature, acrylates/polytrimethylsiloxymethacrylate copolymer. Commercially available products thereof include DOWSII,

FA 4001 CM Silicone Acrylate (30% by mass decamethylcyclopentasiloxane solution), DOWSII, FA 4002 ID Silicone Acrylate (40% by mass isododecane solution), DOWSIL FA 4003 DM Silicone Acrylate (40% by mass dimethicone solution), DOWSIL FA 4004 ID Silicone Acrylate (40% by mass isododecane solution), and DOWSIL FA 4103 Silicone Acrylate Emulsion (30% by mass aqueous solution) (all manufactured by Dow Toray Co., Ltd.).

The acryl-silicone graft copolymer includes a radical polymer of an organopolysiloxane compound having a radical polymerizable group at one terminal of the molecular chain and a radical polymerizable monomer mainly composed of an acrylate and/or a methacrylate.

Examples of the radical polymer of an organopolysiloxane compound having a radical polymerizable group at one terminal of the molecular chain and a radical polymerizable monomer mainly composed of an acrylate and/or a methacrylate usable here include those described in JP H2-25411 A and JP H2-132141 A, and acryl-silicone graft copolymers described in JP H3-162442 A and JP 2003-104825 A.

The acryl-silicone graft copolymer is preferably, as INCI nomenclature, (acrylates/dimethicone) copolymer. Commercially available products thereof include KP-545 (30% by mass decamethylcyclopentasiloxane solution), KP-549 (40% by mass methyltrimethicone solution), and KP-550 (40% by mass isododecane solution) (all manufactured by Shin-Etsu Chemical Co., Ltd.).

The graft-type copolymer or alternate block-type copolymer where a structural unit of a polysiloxane group and a structural unit of a polymer of an unsaturated monomer via a sulfide bond include graft-type copolymers or alternate block-type copolymers described in JP H6-92825 A.

Above all, from the viewpoint of improving film formability and color durability, the component (A2-1) is preferably one or more selected from the group consisting of an acrylic polymer having a carbosiloxane dendrimer structure in the side chain and an acryl-silicone graft copolymer, more preferably one or more selected from (acrylates/polytrimethylsiloxymethacrylate) copolymer and (acrylates/dimethicone) copolymer, and even more preferably (acrylates/dimethicone) copolymer.

[Silicone-Modified Alicyclic Structure-Containing Polymer (A2-2)]

Examples of the silicone-modified alicyclic structure-containing polymer include silicone-modified cyclic polyolefins, and preferred examples thereof include silicone-modified polynorbornenes represented by the following general formula (A2-2-1).

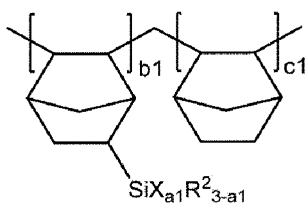

(A2-2-1)

In the formula, each $R^2$ independently represents an alkyl group having 1 or more and 12 or less carbon atoms, X represents a group represented by the following formula (i), a1 is an integer of 1 or more and 3 or less, b1 and c1 each are a repeating unit number, and are each independently an integer of 1 or more.

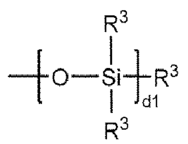

(i)

In the formula, each $R^3$ independently represents a hydrocarbon group having 1 or more and 12 or less carbon atoms, and d1 is an integer of 1 or more and 5 or less.

In the general formula (A2-2-1), $R^2$ is preferably a methyl group, an ethyl group, a n-propyl group, a butyl group, or a pentyl group, and more preferably a methyl group.

X is a group represented by the formula (i), and in the formula (i), each $R^3$ independently is a hydrocarbon group having 1 or more and 12 or less carbon atoms. $R^3$ is preferably an alkyl group having 1 or more and 12 or less carbon atoms or a phenyl group, more preferably an alkyl group having 1 or more and 3 or less carbon atoms, and even more preferably a methyl group. d1 is an integer of 1 or more and 5 or less, and is, from the viewpoint of versatility, preferably d1 = 1. Specifically, X is preferably a trimethylsiloxy group.

a1 is an integer of 1 or more and 3 or less, and, for example, in the polymer, a repeating unit of a1 = 2 and a

repeating unit of a1 = 3 may exist as mixed. From the viewpoint of versatility, a1 is preferably 3.

The proportion of b1 and c1 in the general formula (A2-2-1) is preferably b1/c1 = 20/80 to 90/10 (mol/mol), more preferably 30/70 to 80/20 (mol/mol), and even more preferably 50/50 to 70/30 (mol/mol). The proportion of b1 and c1 can be determined by $^1$H-NMR measurement.

The silicone-modified polynorbornene is preferably a silicone-modified polynorbornene represented by the following formula (A2-2-2).

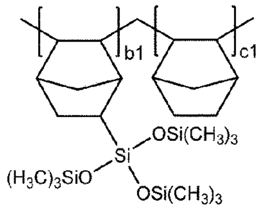

(A2-2-2)

In the formula, b1 and c1 are the same as above.

The silicone-modified polynorbornene represented by the formula (A2-2-2) include a compound of, as INCI nomenclature, (norbornene/tris(trimethylsiloxy)silylnorbornene copolymer).

Commercially available products of the silicone-modified polynorbornene include NBN-30-ID (isododecane solution of (norbornene/tris(trimethylsiloxy)silylnorbomene) copolymer) (by Shin-Etsu Chemical Co., Ltd.).

[Silicone-Modified Pullulan (A2-3)]

The silicone-modified pullulan includes a pullulan having a silicone structure in the side chain, and specifically preferred is a silicone-modified pullulan in which at least a part of the hydrogen atoms of the OH groups in pullulan are substituted with a group represented by the following general formula (ii).

$$—R^4—SiX_{a1}R^2_{3\text{-}a1} \quad \text{(ii)}$$

In the formula, $R^4$ represents a single bond or a divalent organic group, and $R^2$, X and a1 are the same as above. From the viewpoint of versatility, X is preferably a trimethylsiloxyl group, and a1 is preferably 3.

In the general formula (ii), $R^4$ is preferably a divalent organic group, more preferably a divalent group represented by the following general formula (iii) or (iv), and even more preferably a divalent group represented by the general formula (iv).

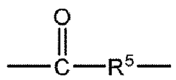

(iii)

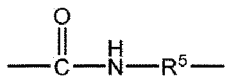

(iv)

In the formulae, $R^5$ represents an alkylene group having 1 or more and 10 or less carbon atoms, and examples thereof include a methylene group, an ethylene group, a trimethylene group, a propylene group and a butylene group. Among these, preferred are an ethylene group, a trimethylene group and a propylene group; and more preferred are a trimethylene group and a propylene group.

Commercially available products of the silicone-modified pullulan include TSPL-30-ID (isododecane solution of tri(trimethylsiloxy)silylpropylcarbamate pullulan), and TSPL-30-D5 (cyclopentasiloxane solution of tri(trimethylsiloxy)silylpropylcarbamate pullulan) (all manufactured by Shin-Etsu Chemical Co., Ltd.).

[PolyurealUrethane Silicone (A2-4)]

The polyurea/urethane silicone of the component (A2-4) includes a polysiloxane/polyurea/polyurethane block terpolymer. For example, it is a dimethylpolysiloxane/urea copolymer of "polyurea-dimethicone" as INCI nomenclature.

The polymer can be produced by copolymerization of an α,ω-aminosilicone and a diisocyanate. Commercially available products of the polyurea/urethane silicone include Wacker-Belsil UD 60, Wacker-Belsil UD 80, Wacker-Belsil

UD 140, and Wacker-Belsil UD 200 (all manufactured by Wacker Corporation).

One or more can be used as the component (A). Among the above, from the viewpoint of improving film formability and color durability, the component (A) preferably contains one or more selected from the group consisting of the component (A1), the component (A2-1), and the component (A2-2), more preferably one or more selected from the group consisting of the component (A1) and the component (A2-1), still more preferably contains one or more selected from the group consisting of the component (A1-2) and the component (A2-1), and even more preferably the component (A1-2).

In addition, both the component (A1) and the component (A2) may be contained as the component (A). In this case, the component (A) preferably includes the component (A1) and one or more selected from the group consisting of the component (A2-1) and the component (A2-2), and more preferably includes the component (A1-2) and the component (A2-1).

More specifically, from the viewpoint of improving film formability, color durability, and the effect of preventing adhesion of dirt, the component (A) preferably contains one or more selected from the group consisting of trimethylsiloxysilicate, phenylpropyldimethylsiloxysilicate, trifluoropropyldimethyltrimethylsiloxysilicate, (trimethylsiloxysilicate/dimethiconol) crosspolymer, polymethylsilsesquioxane, polypropylsilsesquioxane, (acrylates/polytrimethylsiloxymethacrylate) copolymer, (acrylates/dimethicone) copolymer, and (norbornene/tris(trimethylsiloxy)silylnorbomene) copolymer, more preferably contains one or more selected from the group consisting of trimethylsiloxysilicate, phenylpropyldimethylsiloxysilicate, trifluoropropyldimethyltrimethylsiloxysilicate, (trimethylsiloxysilicate/dimethiconol) crosspolymer, polymethylsilsesquioxane, polypropylsilsesquioxane, (acrylates/polytrimethylsiloxymethacrylate) copolymer, and (acrylates/dimethicone) copolymer, still more preferably contains one or more selected from the group consisting of trimethylsiloxysilicate, polymethylsilsesquioxane, and (acrylates/dimethicone) copolymer, even more preferably contains trimethylsiloxysilicate, and even more preferably is trimethylsiloxysilicate.

<Component (B): Organopolysiloxane having a degree of polymerization of 100 or more>

The composition contains an organopolysiloxane having a degree of polymerization of 100 or more as the component (B). It is considered that by performing a step of applying a composition containing the component (B) to an artificially colored or decolored keratin substance or fiber for a head decoration product, an effect of preventing adhesion of dirt can be imparted.

The degree of polymerization of the component (B) is 100 or more, preferably 150 or more, more preferably 250 or more, still more preferably 300 or more, even more preferably 320 or more, and even more preferably 350 or more, from the viewpoint of improving color durability and the effect of preventing adhesion of dirt. On the other hand, from the viewpoint of improving color durability and the effect of preventing adhesion of dirt, and from the viewpoint of availability, the degree of polymerization is preferably 4,300 or less, more preferably 4,200 or less, still more preferably 4,000 or less, even more preferably 3,800 or less, even more preferably 3,700 or less, and even more preferably 3,650 or less. Accordingly, the degree of polymerization of the component (B) is 100 or more, and is preferably 100 or more and 4,300 or less, more preferably 150 or more and 4,200 or less, still more preferably 250 or more and 4,200 or less, even more preferably 300 or more and 4,000 or less, even more preferably 320 or more and 3,800 or less, even more preferably 320 or more and 3,700 or less, and even more preferably 350 or more and 3,650 or less.

More specifically, the component (B) is preferably an organopolysiloxane represented by the following general formula (1).

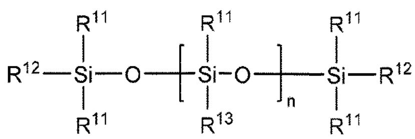
(1)

In the formula, each $R^{11}$ independently represents a hydrocarbon group having 1 or more and 6 or less carbon atoms, each $R^{12}$ independently represents a hydroxy group, an alkoxy group having 1 or more and 6 or less carbon atoms, or a hydrocarbon group having 1 or more and 6 or less carbon atoms, $R^{13}$ represents a hydrocarbon group having 1 or more and 6 or less carbon atoms or a primary to tertiary amino group-containing group, n represents a degree of polymerization and is a number of 100 or more, and n's $R^{13}$s may be the same or different from each other.

In the general formula (1), the hydrocarbon group in $R^{11}$ may be either an aliphatic group or an aromatic group, and examples thereof include an alkyl group, an alkenyl group, and a phenyl group. The alkyl group and the alkenyl group may be linear or branched.

Among the above, $R^{11}$ is preferably an alkyl group having 1 or more and 6 or less carbon atoms or a phenyl group, more preferably an alkyl group having 1 or more and 3 or less carbon atoms or a phenyl group, still more preferably

a methyl group or a phenyl group, and even more preferably a methyl group.

In the general formula (1), each $R^{12}$ independently represents a hydroxy group, an alkoxy group having 1 or more and 6 or less carbon atoms, or a hydrocarbon group having 1 or more and 6 or less carbon atoms.

Examples of the alkoxy group in $R^{12}$ include a methoxy group, an ethoxy group, a propoxy group, and a butoxy group.

The hydrocarbon group in $R^{12}$ is the same as in $R^{11}$, and is preferably an alkyl group having 1 or more and 6 or less carbon atoms or a phenyl group, more preferably an alkyl group having 1 or more and 3 or less carbon atoms or a phenyl group, still more preferably a methyl group or a phenyl group, and even more preferably a methyl group.

From the viewpoint of improving the effect of preventing adhesion of dirt, $R^{12}$ is preferably a hydroxy group, an alkyl group having 1 or more and 6 or less carbon atoms, or a phenyl group, more preferably a hydroxy group, an alkyl group having 1 or more and 3 or less carbon atoms, or a phenyl group, still more preferably a hydroxy group, a methyl group, or a phenyl group, and even more preferably a methyl group.

In the general formula (1), $R^{13}$ represents a hydrocarbon group having 1 or more and 6 or less carbon atoms or a primary to tertiary amino group-containing group.

The hydrocarbon group in $R^{13}$ is the same as in $R^{11}$, and is preferably an alkyl group having 1 or more and 6 or less carbon atoms or a phenyl group, more preferably an alkyl group having 1 or more and 3 or less carbon atoms or a phenyl group, still more preferably a methyl group or a phenyl group, and even more preferably a methyl group.

The primary to tertiary amino group-containing group in $R^{13}$ (hereinafter, also simply referred to as "amino group-containing group") is preferably a group represented by $-N(R^{14})_2$, $-NR^{14}(CH_2)_pN(R^{14})_2$, or $-NR^{14}(CH_2)_pN(R^{15})CO-R^{16}$. Here, $R^{14}$ represents a hydrogen atom or a monovalent hydrocarbon group having 1 or more and 4 or less carbon atoms, and preferably a hydrogen atom, a methyl group or an ethyl group. $R^{15}$ represents a monovalent hydrocarbon group having 1 or more and 4 or less carbon atoms, and is preferably a methyl group or an ethyl group. $R^{16}$ represents a monovalent hydrocarbon group having 1 or more and 4 or less carbon atoms, p is a number of 2 or more and 6 or less, and is preferably a number of 2 or more and 4 or less.

The amino group-containing group in $R^{13}$ is preferably $-(CH_2)_3-NH_2$, $-(CH_2)_3-N(CH_3)_2$, $-(CH_2)_3-NH-(CH_2)_2-NH_2$, or $-(CH_2)_2-NH-(CH_2)_2-N(CH_3)_2$, and more preferably $-(CH_2)_3-NH_2$.

$R^{13}$ is preferably a hydrocarbon group having 1 or more and 6 or less carbon atoms, more preferably an alkyl group having 1 or more and 6 or less carbon atoms or a phenyl group, still more preferably an alkyl group having 1 or more and 3 or less carbon atoms or a phenyl group, even more preferably a methyl group or a phenyl group, and even more preferably a methyl group, from the viewpoint of improving the effect of preventing adhesion of dirt.

In the general formula (1), n represents a degree of polymerization, and from the viewpoint of improving color durability and the effect of preventing adhesion of dirt, n is a number of 100 or more, and is preferably 150 or more, more preferably 250 or more, still more preferably 300 or more, even more preferably 320 or more, and even more preferably 350 or more. On the other hand, from the viewpoint of improving color durability and the effect of preventing adhesion of dirt, and from the viewpoint of availability, the degree of polymerization is preferably 4,300 or less, more preferably 4,200 or less, still more preferably 4,000 or less, even more preferably 3,800 or less, even more preferably 3,700 or less, and even more preferably 3,650 or less. Accordingly, n of the general formula (1) is 100 or more, and is preferably 100 or more and 4,300 or less, more preferably 150 or more and 4,200 or less, still more preferably 250 or more and 4,200 or less, even more preferably 300 or more and 4,000 or less, even more preferably 320 or more and 3,800 or less, even more preferably 320 or more and 3,700 or less, and even more preferably 350 or more and 3,650 or less.

The viscosity at 25°C of the component (B) is preferably 120 $mm^2/s$ or more, more preferably 200 $mm^2/s$ or more, still more preferably 500 $mm^2/s$ or more, even more preferably 700 $mm^2/s$ or more, even more preferably 800 $mm^2/s$ or more, and even more preferably 1,000 $mm^2/s$ or more, from the viewpoint of improving color durability and the effect of preventing adhesion of dirt. On the other hand, from the viewpoint of improving color durability and the effect of preventing adhesion of dirt, and from the viewpoint of availability, the viscosity at 25°C of the component (B) is preferably 100,000,000 $mm^2/s$ or less, more preferably 80,000,000 $mm^2/s$ or less, still more preferably 50,000,000 $mm^2/s$ or less, even more preferably 40,000,000 $mm^2/s$ or less, even more preferably 35,000,000 $mm^2/s$ or less, even more preferably 30,000,000 $mm^2/s$ or less, and even more preferably 25,000,000 $mm^2/s$ or less.

Therefore, the viscosity at 25°C of the component (B) is preferably 120 $mm^2/s$ or more and 100,000,000 $mm^2/s$ or less, more preferably 200 $mm^2/s$ or more and 80,000,000 $mm^2/s$ or less, still more preferably 500 $mm^2/s$ or more and 80,000,000 $mm^2/s$ or less, even more preferably 700 $mm^2/s$ or more and 80,000,000 $mm^2/s$ or less, even more preferably 700 $mm^2/s$ or more and 50,000,000 $mm^2/s$ or less, even more preferably 800 $mm^2/s$ or more and 40,000,000 $mm^2/s$ or less, even more preferably 800 $mm^2/s$ or more and 35,000,000 $mm^2/s$ or less, even more preferably 800 $mm^2/s$ or more and 30,000,000 $mm^2/s$ or less, and even more preferably 1,000 $mm^2/s$ or more and 25,000,000 $mm^2/s$ or less.

The viscosity is a value measured at 25°C based on JIS Z 8803:2011 "Methods for viscosity measurement of liquid", and can be measured by selecting an appropriate one from a capillary viscometer, a falling ball viscometer, a rotary viscometer, and a vibration viscometer. In addition, when the viscosity exceeds the common measurement range of a viscometer, it can be obtained from a diluted solution of the component (B) by the following method.

A toluene solution of the component (B) having a concentration of 1 g/100 mL is prepared, and the specific viscosity ηsp (25°C) is determined by the following equation (1). Next, the intrinsic viscosity [η] is determined by substituting the relationship of Huggins shown in the following equation (2). Further, [η] is substituted into the A. Kolorlov equation shown in the following equation (3) to determine the molecular weight M. Finally, M is substituted into the A. J. Barry equation shown in the following equation (4) to determine the viscosity η of the component (B) (see, for example, Silicone Oil KF-96 Performance Test Result 4.2, published by Shin-Etsu Chemical Co., Ltd.).

$$\eta sp = (\eta/\eta 0) - 1 \quad (1)$$

(η0: viscosity of toluene, η: viscosity of solution)

$$\eta sp = [\eta] + K'[\eta]^2 \quad (2)$$

(K': Huggins constant, and the one described in Nakamuta, Nihon Kagakukai-shi, 77588 [1956] is used.)

$$[\eta] = 0.215 \times 10^{-4} M^{0.65} \quad (3)$$

$$\log\eta = 1.00 + 0.0123 M^{0.5} \quad (4)$$

Examples of a commercially available product of dimethylpolysiloxane used as the component (B) include KF-96-1,000cs (viscosity of 1,000 $mm^2/s$), KF-96H-10000cs (viscosity of 10,000 $mm^2/s$), KF-96H-100000cs (viscosity of 100,000 $mm^2/s$), KF-96H-300000cs (viscosity of 300,000 $mm^2/s$), KF-96H-500000cs (viscosity of 500,000 $mm^2/s$), X-21-5686 (viscosity of 3,000,000 $mm^2/s$), and X-25-9074 (viscosity of 30,000,000 $mm^2/s$) (all manufactured by Shin-Etsu Chemical Co., Ltd.), and Silsoft B3020 (viscosity of 20,000,000 $mm^2/s$) (manufactured by Momentive Performance Materials, Inc.).

Examples of a commercially available product of aminopropylmethylpolysiloxane used as the component (B) include KF-8017 (10% by mass - low viscosity dimethylpolysiloxane solution), KF-8018 (10% by mass - cyclopentasiloxane solution), and KF-8020 (20% by mass - low viscosity dimethylpolysiloxane solution) (all manufactured by Shin-Etsu Chemical Co., Ltd.).

Examples of a commercially available product of dimethiconol used as the component (B) include X-21-5613 (20% by mass - low viscosity dimethylpolysiloxane solution), X-21-5666 (30% by mass - cyclopentasiloxane solution), X-21-5847, and X-21-5849 (all manufactured by Shin-Etsu Chemical Co., Ltd.).

From the viewpoint of improving the effect of preventing adhesion of dirt, the component (B) is preferably one or more selected from the group consisting of dimethylpolysiloxane, methylphenylpolysiloxane, aminopropylmethylpolysiloxane, and dimethiconol having a degree of polymerization within the above range, and more preferably dimethylpolysiloxane having a degree of polymerization within the above range.

(Content)

The content of the component (A) in the composition is preferably 0.1% by mass or more, more preferably 0.5% by mass or more, still more preferably 1% by mass or more, even more preferably 2% by mass or more, and even more preferably 3% by mass or more, from the viewpoint of improving film formability, color durability, and the effect of preventing adhesion of dirt, and is preferably 30% by mass or less, more preferably less than 25% by mass, still more preferably 20% by mass or less, and even more preferably 15% by mass or less, from the viewpoint of improving color durability. Therefore, the content of the component (A) in the composition is preferably 0.1% by mass or more and 30% by mass or less, more preferably 0.5% by mass or more and 25% by mass or less, still more preferably 1% by mass or more and 20% by mass or less, even more preferably 2% by mass or more and 15% by mass or less, and even more preferably 3% by mass or more and 15% by mass or less.

The content of the component (B) in the composition is preferably 0.1% by mass or more, more preferably 0.3% by mass or more, and still more preferably 0.5% by mass or more, from the viewpoint of improving film formability, color durability, and the effect of preventing adhesion of dirt, and is preferably 30% by mass or less, more preferably 25% by mass or less, still more preferably 20% by mass or less, even more preferably 15% by mass or less, and even more preferably 10% by mass or less, from the viewpoint of improving the effect of preventing adhesion of dirt. Thus, the content of the component (B) in the composition is preferably 0.1% by mass or more and 30% by mass or less, more

preferably 0.3% by mass or more and 25% by mass or less, still more preferably 0.5% by mass or more and 20% by mass or less, even more preferably 0.5% by mass or more and 15% by mass or less, and even more preferably 0.5% by mass or more and 10% by mass or less.

The total content of the component (A) and the component (B) in the composition is preferably 0.2% by mass or more, more preferably 0.5% by mass or more, still more preferably 1% by mass or more, even more preferably 2% by mass or more, even more preferably 3% by mass or more, even more preferably 3.5% by mass or more, and even more preferably 5% by mass or more from the viewpoint of improving film formability, color durability, and the effect of preventing adhesion of dirt. On the other hand, from the viewpoint of improving the effect of preventing adhesion of dirt, the total content thereof is preferably 50% by mass or less, more preferably 45% by mass or less, still more preferably 40% by mass or less, even more preferably 35% by mass or less, even more preferably 25% by mass or less, and even more preferably 20% by mass or less. Thus, the total content of the component (A) and the component (B) in the composition is preferably 0.2% by mass or more and 50% by mass or less, more preferably 0.5% by mass or more and 45% by mass or less, still more preferably 1% by mass or more and 40% by mass or less, even more preferably 1% by mass or more and 35% by mass or less, even more preferably 2% by mass or more and 35% by mass or less, even more preferably 2% by mass or more and 25% by mass or less, even more preferably 3% by mass or more and 25% by mass or less, even more preferably 3.5% by mass or more and 25 by mass or less, and even more preferably 5% by mass or more and 20% by mass or less.

In addition, the proportion of the content mass of the component (A) to the total content mass of the component (A) and the component (B) [(A)/ f (A) + (B)}] in the composition is 10% or more, and is preferably 20% or more, more preferably 30% or more, still more preferably 40% or more, and even more preferably 50% or more, from the viewpoint of improving film formability, color durability, and the effect of preventing adhesion of dirt. On the other hand, from the viewpoint of improving color durability, the proportion is preferably 98% or less, and more preferably 95% or less. Thus, the proportion of the content mass of the component (A) to the total content mass of the component (A) and the component (B) [(A)/{ (A) + (B)}] in the composition is 10% or more, and is preferably 10% or more and 98% or less, more preferably 10% or more and 95% or less, still more preferably 20% or more and 95% or less, even more preferably 30% or more and 95% or less, even more preferably 40% or more and 95% or less, and even more preferably 50% or more and 95% or less.

(Component (C): Organopolysiloxane other than component (A) and component (B), which has a polyalkylene oxide moiety and a cationic group)

From the viewpoint of improving color durability and the effect of preventing adhesion of dirt, the composition may further contain, as a component (C), an organopolysiloxane other than the component (A) and the component (B), which has a polyalkylene oxide moiety and a cationic group. Since the component (C) has high adsorptivity to the keratin substance or the fiber for a head decoration product having a negative charge, it is considered that the component (C) is unevenly distributed on the keratin substance or the fiber for a head decoration product side in the film formed, strongly adheres the film to the surface thereof, and enhances the peeling prevention effect of the film.

The cationic group contained in the component (C) refers to a cationic group or a group which can be ionized to become a cationic group. Specific examples thereof include a primary amino group, a secondary amino group, a tertiary amino group, and a quaternary ammonium group, and from the viewpoint of adsorptivity to the surface of the keratin substance or the fiber for a head decoration product, the cationic group is preferably one or more selected from the group consisting of a primary amino group, a secondary amino group, and a tertiary amino group.

The number of carbon atoms of the alkylene oxide constituting the polyalkylene oxide moiety of the component (C) may be 1 or more, and from the viewpoint of availability, it is preferably 2 or more, and is preferably 6 or less, more preferably 4 or less, and still more preferably 3 or less.

The alkylene oxide constituting the polyalkylene oxide moiety may be one kind or two or more kinds.

Specific examples of the alkylene oxide include one or more selected from the group consisting of ethylene oxide, propylene oxide, trimethylene oxide, butylene oxide, tetramethylene oxide, pentamethylene oxide, and hexamethylene oxide. Among these, from the viewpoint of availability, the alkylene oxide constituting the polyalkylene oxide moiety is preferably one or more selected from the group consisting of ethylene oxide, propylene oxide, trimethylene oxide, butylene oxide, and tetramethylene oxide, more preferably one or more selected from the group consisting of ethylene oxide, propylene oxide, and trimethylene oxide, still more preferably one or more selected from the group consisting of ethylene oxide and propylene oxide, and even more preferably ethylene oxide from the viewpoint of localizing the component (C) in the formed film on the side of the keratin substance or the fiber for a head decoration product.

The average addition mole number of alkylene oxide in the polyalkylene oxide moiety is not particularly limited, but is preferably 2 or more, more preferably 4 or more, and still more preferably 10 or more. From the viewpoint of availability, it is preferably 100 or less, more preferably 80 or less, and still more preferably 50 or less.

In the component (C), the polyalkylene oxide moiety and the cationic group may be present in the organosiloxane

main chain, or may be present in a side chain portion.

More specifically, the component (C) is more preferably one or more selected from the group consisting of an aminopolyether-modified silicone (C1) having a repeating unit represented by the general formula (2) shown below and an aminopolyether-modified silicone (C2) having a repeating unit represented by the general formula (3) shown below.

In the following description, the aminopolyether-modified silicone (C1) having a repeating unit represented by the general formula (2) is also referred to as "component (C1)", and the aminopolyether-modified silicone (C2) having a repeating unit represented by the general formula (3) is also referred to as "component (C2)".

[Component (C1)]

The component (C1) is an aminopolyether-modified silicone having a repeating unit represented by the following general formula (2).

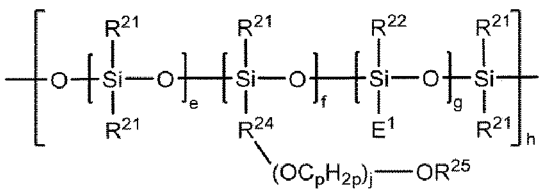

(2)

In the formula (2), $R^{21}$ represents a monovalent hydrocarbon group having 1 or more and 6 or less carbon atoms, $R^{22}$ represents any of $R^{21}$ or $E^1$, $E^1$ represents a monovalent group represented by $-R^{23}-Z^1$ (where $R^{23}$ represents a divalent hydrocarbon group having 1 or more and 6 or less carbon atoms, and $Z^1$ represents a primary to tertiary amino group-containing group), $R^{24}$ represents a divalent hydrocarbon group having 1 or more and 6 or less carbon atoms, $R^{25}$ represents a hydrogen atom or a monovalent hydrocarbon group having 1 or more and 4 or less carbon atoms.

e is a number of 1 or more and 50 or less, f is a number of 1 or more and 50 or less, g is a number of 1 or more and 50 or less, h is a number of 1 or more, j is a number of 2 or more and 100 or less, p is a number of 2 or more and 10 or less, the bonding order of the parenthesized structural units is not limited, and the bonding form thereof may be in a block form or a random form, j's $(OC_pH_{2p})$s can be the same or different, and plural $R^{21}$'s, $R^{22}$'s, $R^{24}$'s, $R^{25}$'s and $E^1$'s can be the same or different.

In the general formula (2), $R^{21}$ represents a monovalent hydrocarbon group having 1 or more and 6 or less carbon atoms, and is preferably an alkyl group having 1 or more and 6 or less carbon atoms, or a phenyl group, more preferably a methyl group or an ethyl group, and even more preferably a methyl group. $R^{22}$ is any of $R^{21}$ or $E^1$, and is preferably $R^{21}$.

In the general formula (2), $E^1$ represents a monovalent group represented by $-R^{23}-Z'$ (where $R^{23}$ represents a divalent hydrocarbon group having 1 or more and 6 or less carbon atoms, and $Z^1$ represents a primary to tertiary amino group-containing group). $R^{23}$ is preferably a divalent hydrocarbon group having 2 or more and 4 or less carbon atoms, and more preferably an ethylene group, a trimethylene group, a propylene group or a tetramethylene group.

$Z^1$ is a primary to tertiary amino group-containing group, and is preferably a group represented by $-N(R^{26})_2$, $-NR^{26}(CH_2)_qN(R^{26})_2$, or $-NR^{26}(CH_2)_qN(R^{27})CO-R^{28}$. Here, $R^{26}$ represents a hydrogen atom or a monovalent hydrocarbon group having 1 or more and 4 or less carbon atoms, and preferably a hydrogen atom, a methyl group or an ethyl group. $R^{27}$ represents a monovalent hydrocarbon group having 1 or more and 4 or less carbon atoms, and is preferably a methyl group or an ethyl group. $R^{28}$ represents a monovalent hydrocarbon group having 1 or more and 4 or less carbon atoms. q is a number of 2 or more and 6 or less, and is preferably a number of 2 or more and 4 or less.

In the general formula (2), the group $E^1$ is preferably $-(CH_2)_3-NH_2$, $-(CH_2)_3-N(CH_3)_2$, $-(CH_2)_3-NH-(CH_2)_2-NH_2$, or $-(CH_2)_2-NH-(CH_2)_2-N(CH_3)_2$, and more preferably $-(CH_2)_3-NH_2$, or $-(CH_2)_3-NH-(CH_2)_2-NH_2$.

In the general formula (2), $R^{24}$ is a divalent hydrocarbon group having 1 or more and 6 or less carbon atoms, preferably a divalent hydrocarbon group having 2 or more and 4 or less carbon atoms, and more preferably an ethylene group, a trimethylene group, a propylene group or a tetramethylene group.

$R^{25}$ is a hydrogen atom or a monovalent hydrocarbon group having 1 or more and 4 or less carbon atoms, and is preferably a methyl group or an ethyl group.

In the general formula (2), e is a number of 1 or more and 50 or less, f is a number of 1 or more and 50 or less, g is a number of 1 or more and 50 or less, h is a number of 1 or more, and j is a number of 2 or more and 100 or less. p is a number of 2 or more and 10 or less, and is preferably 2 or more and 6 or less, and more preferably 2 or more and 4 or less.

In particular, the component (C1) is more preferably one represented by the following general formula (2-1).

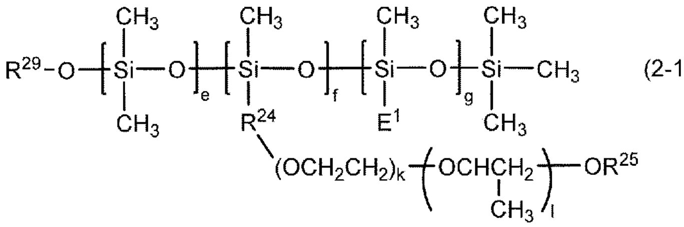

(2-1)

In the formula (2-1), $E^1$, $R^{24}$, $R^{25}$, e, f, and g are the same as above, $R^{29}$ represents a monovalent hydrocarbon group having 1 or more and 4 or less carbon atoms, or a trimethylsilyl group, k is a number of 1 or more and 50 or less, l is a number of 0 or more and 50 or less, and is preferably a number of 1 or more and 50 or less.

$R^{29}$ is preferably a methyl group, an ethyl group or a trimethylsilyl group, and more preferably a trimethylsilyl group.

As the component (C1), one or more can be used either singly or as combined.

As the component (C1), commercially-available aminopolyether-modified silicones can be used. Examples thereof include ABIL Soft AF 100 (aminopolyether-modified silicone represented by the general formula (2-1) (methoxy PEG/PPG-7/3 aminopropyl dimethicone)) (manufactured by Evonik Corporation).

[Component (C2)]

The component (C2) is an aminopolyether-modified silicone having a repeating unit represented by the following general formula (3).

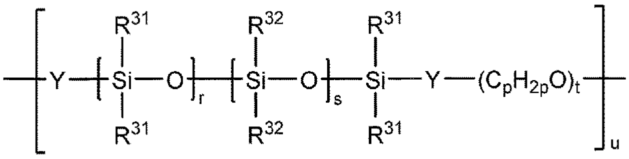

(3)

In the formula (3), $R^{31}$ represents a monovalent hydrocarbon group having 1 or more and 6 or less carbon atoms, $R^{32}$ represents any of $R^{31}$ or $E^2$, $E^2$ represents a monovalent group represented by $-R^{33}-Z^2$ (where $R^{33}$ represents a single bond, or a divalent hydrocarbon group having 1 or more and 20 or less carbon atoms, and $Z^2$ represents a primary to tertiary amino group-containing group). Y represents a single bond, or a divalent group having 1 or more and 12 or less carbon atoms. In the case where all $R^{32}$'s are $R^{31}$'s, at least one Y is an amino group-containing divalent group. In the case where all Y's are divalent groups not containing an amino group, at least one $R^{32}$ is $E^2$.

r is a number of 1 or more, s is a number of 1 or more, t is a number of 2 or more and 100 or less, and u is a number of 1 or more. p is the same as above, and is a number of 2 or more and 10 or less. The bonding order of the parenthesized structural units is not limited, and the bonding form thereof may be in a block form or a random form, t's $(C_pH_{2p}O)$s can be the same or different. Plural $R^{31}$'s, $R^{32}$'s, $E^2$'s and Y's can be the same or different.

In the general formula (3), $R^{31}$ represents a monovalent hydrocarbon group having 1 or more and 6 or less carbon atoms, and is independently preferably an alkyl group having 1 or more and 6 or less carbon atoms, or a phenyl group, more preferably a methyl group or an ethyl group, and still more preferably a methyl group.

In the general formula (3), $R^{32}$ is any of $R^{31}$ or $E^2$. $E^2$ represents a monovalent group represented by $-R^{33}-Z^2$ (where $R^{33}$ represents a single bond, or a divalent hydrocarbon group having 1 or more and 20 or less carbon atoms).

$R^{33}$ is preferably a divalent hydrocarbon group having 1 or more and 20 or less carbon atoms, more preferably an alkylene group having 1 or more and 20 or less carbon atoms, still more preferably a linear or branched alkylene group having 1 or more and 6 or less carbon atoms, even more preferably a methylene group, an ethylene group, a trimethylene group, a propylene group, a tetramethylene group, or a hexamethylene group, and even more preferably a trimethylene group or a propylene group.

$Z^2$ is a primary to tertiary amino group-containing group, and is preferably an amino group-containing group represented by $-N(R^{34})_2$, $-NR^{34}(CH_2)_qN(R^{34})_2$, or $-NR^{34}(CH_2)_qN(R^{35})CO-R^{36}$. Here, $R^{34}$ and $R^{35}$ each independently represent a hydrogen atom or an alkyl group having 1 or more and 3 or less carbon atoms, and is preferably a hydrogen atom or a methyl group. $R^{36}$ represents an alkyl group having 1 or more and 3 or less carbon atoms. q is a number of 1 or more and 6 or less, and is preferably a number of 2 or more and 4 or less.

In the general formula (3), the group $E^2$ is preferably $-(CH_2)_3-NH_2$, $-(CH_2)_3-N(CH_3)_2$, $-(CH_2)_3-NH-(CH_2)_2-NH_2$, or $-(CH_2)_2-NH-(CH_2)_2-N(CH_3)_2$, and more preferably $-(CH_2)_3-NH_2$, or $-(CH_2)_3-NH-(CH_2)_2-NH_2$.

In the general formula (3), Y is a single bond, or a divalent group having 1 or more and 12 or less carbon atoms. The divalent group having 1 or more and 12 or less carbon atoms is preferably an alkylene group having 1 or more and 6 or less carbon atoms, an alkyleneoxy group having 1 or more and 6 or less carbon atoms, or a divalent group represented by $-R^{37}-O-CH_2-CH(OH)-CH_2-N(R^{38})-R^{39}-O-$ of an amino group-containing divalent group. Here, $R^{37}$ and $R^{39}$ each independently represent an alkylene group having 1 or more and 6 or less carbon atoms, preferably an alkylene group having 2 or more and 4 or less carbon atoms, and more preferably a propylene group. $R^{38}$ represents a hydrogen atom or an alkyl group having 1 or more and 3 or less carbon atoms.

In the general formula (3), the alkylene group having 1 or more and 6 or less carbon atoms and the alkylene group in the alkyleneoxy group having 1 or more and 6 or less carbon atoms for Y are preferably an ethylene group, a propylene group, a trimethylene group, a n-butylene group (tetramethylene group) or an isobutylene group, and more preferably a n-butylene group or an isobutylene group. The isobutylene group as referred to herein includes $-CH(CH_3)CH_2CH_2-$, $-CH_2CH(CH_3)CH_2-$, and $-CH_2CH_2CH(CH_3)-$.

In the general formula (3), r is a number of 1 or more, s is a number of 1 or more, t is a number of 2 or more and 100 or less, and u is a number of 1 or more. r is preferably a number of 1 or more and 1000 or less, and more preferably a number of 2 or more and 200 or less. s is preferably a number of 1 or more and 100 or less, t is preferably a number of 4 or more and 80 or less, and more preferably 10 or more and 50 or less, u is preferably a number of 1 or more and 300 or less, and more preferably a number of 1 or more and 150 or less.

In the general formula (3), p is a number of 2 or more and 10 or less, preferably 2 or more and 6 or less, and more preferably 2 or more and 4 or less.

The component (C2) is more preferably one or more selected from the group consisting of an aminopolyether-modified silicone having a structure represented by the following general formula (3-1) and an aminopolyether-modified silicone having a structure represented by the following general formula (3-2).

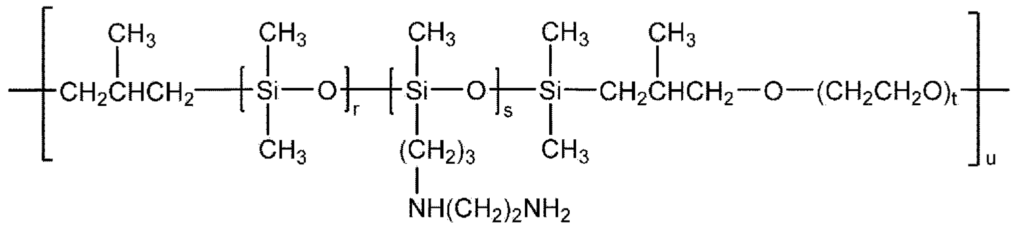

(3-1)

In the formula (3-1), r, s, t and u are the same as above.

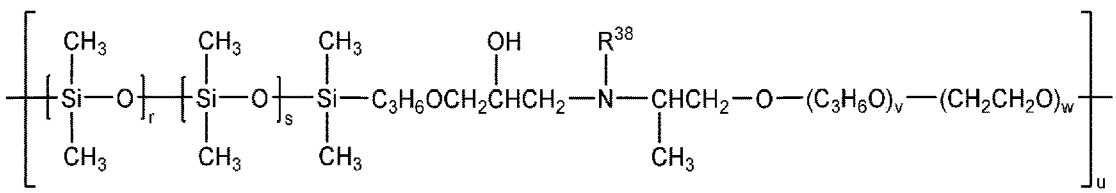

(3-2)

In the formula (3-2), $R^{38}$, r, s and u are the same as above, v is a number of 0 or more and 50 or less, and preferably a number of 2 or more and 50 or less. w is a number of 2 or more and 100 or less. v + w is a number of 2 or more and 100 or less, preferably a number of 4 or more and 80 or less, and more preferably a number of 10 or more and 50 or less.

As the component (C2), one or more can be used either singly or as combined.

As the component (C2), commercially-available aminopolyether-modified silicones can be used. Examples thereof include, as the aminopolyether-modified silicone having a structure represented by the general formula (3-1), DOWSIL SS-3588 Fluid (80% by mass ethanol solution of (bisisobutyl-PEG-15/amodimethicone) copolymer), DOWSIL SILSTYLE 104 ((bisisobutyl-PEG-14/amodimethicone) copolymer), DOWSIL SILSTYLE 201 ((bisisobutyl-PEG-14/amodimethicone) copolymer), and DOWSIL SILSTYLE 401((bisisobutyl PEG/PPG-20/35/amodimethicone) copolymer) (all manufactured by Dow Toray Co., Ltd.). In addition, examples of the aminopolyether-modified silicone having a structure represented by the general formula (3-2) include Silsoft A+ (PEG-40/PPG-8 methylaminopropyl/hydroxypropyldimethicone copolymer) (manufactured by Momentive Performance Materials, Inc.).

As the component (C), one or more of the above can be used. Among these, from the viewpoint of adsorptivity to the surface of the keratin substance or the fiber for a head decoration product, the component (C) is preferably an aminopolyether-modified silicone (C2) having a repeating unit represented by the general formula (3), more preferably

one or more selected from the group consisting of an aminopolyether-modified silicone having a structure represented by the general formula (3-1) and an aminopolyether-modified silicone having a structure represented by the general formula (3-2), still more preferably an aminopolyether-modified silicone having a structure represented by the general formula (3-1), and even more preferably an aminopolyether-modified silicone composed of a structure represented by the general formula (3-1).

When the composition contains the component (C), the content of the component (C) in the composition is preferably 0.01% by mass or more, more preferably 0.1% by mass or more, still more preferably 0.5% by mass or more, even more preferably 0.6% by mass or more, and even more preferably 0.7% by mass or more, and is preferably 25% by mass or less, more preferably 15% by mass or less, still more preferably 12% by mass or less, even more preferably 10% by mass or less, and even more preferably 5% by mass or less, from the viewpoint of improving adsorptivity to the surface of the keratin substance or the fiber for a head decoration product to enhance the effect of preventing peeling of a film and improving color durability and the effect of preventing adhesion of dirt. Thus, the content of the component (C) in the composition is preferably 0.01% by mass or more and 25% by mass or less, more preferably 0.1% by mass or more and 15% by mass or less, still more preferably 0.5% by mass or more and 12% by mass or less, even more preferably 0.6% by mass or more and 10% by mass or less, and even more preferably 0.7% by mass or more and 5% by mass or less.

(Component (D): Solvent)

The composition preferably further contains a solvent as a component (D) from the viewpoint of dissolving or dispersing the component (A), the component (B), and other components, and from the viewpoint of adjusting the viscosity to a viscosity that is easy to apply to the keratin substance or the fiber for a head decoration product.

As the solvent, a liquid organic solvent is preferable from the viewpoint of easy handling, and examples thereof include alcohol-based solvents, ether-based solvents, ketone-based solvents, ester-based solvents, hydrocarbon-based solvents, and silicone-based solvents, and the solvent can be appropriately selected according to the formulation. Among these, from the viewpoint of improving usability after drying, it is preferable to include a volatile solvent.

Among the volatile solvents, examples of the alcohol-based solvent include ethanol, 1-propanol, 2-propanol, 1-butanol, 2-butanol, and benzyl alcohol; examples of the ether-based solvent include diethyl ether and tetrahydrofuran; examples of the ketone-based solvent include acetone and methyl ethyl ketone; examples of the ester-based solvent include methyl acetate, ethyl acetate, butyl acetate, and isobutyl acetate; examples of the hydrocarbon-based solvent include light liquid isoparaffin (containing isoparaffin having 8 to 16 carbon atoms as a main component), pentane, isopentane, hexane, isohexane, heptane, isoheptane, decane, isodecane, dodecane, isododecane, tridecane, isotridecane, tetradecane, and isotetradecane; and examples of the silicone-based solvent include cyclic silicone such as decamethylcyclopentasiloxane, dimethylpolysiloxane having a viscosity at 25°C of 4 $mm^2/s$ or less, and alkyl trimethicone such as methyl trimethicone. One or more of these solvents can be used.

From the viewpoint of the solubility of the component (A) and the component (B), the component (D) preferably contains one or more solvents selected from the group consisting of dimethylpolysiloxane having a viscosity at 25°C of 4 $mm^2/s$ or less, methyltrimethicone, pentane, isopentane, hexane, isohexane, heptane, isoheptane, decane, isodecane, dodecane, isododecane, tridecane, isotridecane, tetradecane, isotetradecane, and light liquid isoparaffin, more preferably one or more solvents selected from the group consisting of dimethylpolysiloxane having a viscosity at 25°C of 4 $mm^2/s$ or less, methyltrimethicone, isodecane, isododecane, isotetradecane, and light liquid isoparaffin, and still more preferably one or more solvents selected from the group consisting of isodecane, isododecane, isotetradecane, and light liquid isoparaffin.

When the composition contains the component (D), the content thereof is preferably 40% by mass or more, more preferably 50% by mass or more, and still more preferably 60% by mass or more from the viewpoint of dissolving or dispersing the component (A), the component (B), and other components, and is preferably 99% by mass or less, and more preferably 98% by mass or less from the viewpoint of adjusting the viscosity to a viscosity that is easy to apply to the keratin substance or the fiber for a head decoration product. Accordingly, the content of the component (D) in the composition is preferably 40% by mass or more and 99% by mass or less, more preferably 50% by mass or more and 99% by mass or less, and still more preferably 60% by mass or more and 98% by mass or less.

In addition to the components (A) to (D), the composition can contain components usually used in a cosmetic composition, for example, a functional powder, an oil agent other than the above-described components, an antioxidant, a perfume, a pigment, a dye, an antiseptic, a viscosity improver, a pH modifier, a blood circulation promoting agent, a cooling agent, an antiperspirant, a bactericide, a skin activating agent, a moisturizing agent, and a refreshing agent.

The method for producing the composition is not particularly limited, and the first agent can be produced according to a conventional method.

The composition preferably has a low content of water from the viewpoint of improving color durability and the effect of preventing adhesion of dirt. The content of water is preferably 10% by mass or less, more preferably less than

5% by mass, and still more preferably less than 2% by mass in the composition.

The composition preferably has a low content of polysilicone-polyamide copolymer from the viewpoint of improving color durability and the effect of preventing adhesion of dirt. The content of the polysilicone-polyamide copolymer is preferably less than 5% by mass, more preferably less than 2% by mass, still more preferably less than 1% by mass, even more preferably less than 0.5% by mass, even more preferably less than 0.1% by mass, and even more preferably 0% by mass in the composition.

The composition preferably has a low content of solid oil from the viewpoint of improving color durability and the effect of preventing adhesion of dirt. The solid oil is an oil agent that is solid at 25°C, and examples thereof include paraffin-based waxes such as solid paraffin, polyolefin-based waxes such as polyethylene wax, and beeswax. The content of the solid oil is preferably less than 50% by mass, more preferably less than 20% by mass, still more preferably less than 10% by mass, even more preferably less than 5% by mass, and even more preferably less than 1% by mass in the composition.

The composition preferably has a low content of a nonvolatile liquid oil agent other than the components (B) and (C) from the viewpoint of improving color durability and the effect of preventing adhesion of dirt. The nonvolatile liquid oil agent is an oil agent that has a boiling point higher than 260°C at atmospheric pressure and is liquid at 25°C, and examples thereof include nonvolatile hydrocarbon oils such as liquid isoparaffin; nonvolatile liquid silicones other than the components (B) and (C); triglycerides such as glyceryl trioctanoate, glyceryl tri-2-ethylhexanoate, caprylic/capric triglyceride, avocado oil, olive oil, sesame seed oil, rice bran oil, safflower oil, soybean oil, corn oil, rapeseed oil, castor oil, cotton seed oil, and mink oil; fatty acids such as oleic acid and isostearic acid; ester oils such as isopropyl myristate, butyl myristate, isopropyl palmitate, ethyl oleate, ethyl linoleate, isopropyl linoleate, cetyl caprylate, hexyl laurate, decyl myristate, decyl oleate, oleyl oleate, isostearyl laurate, isotridecyl myristate, isocetyl myristate, isostearyl myristate, octyldodecyl myristate, octyl palmitate, isocetyl palmitate, isostearyl palmitate, isodecyl oleate, isopropyl isostearate, cetyl 2-ethylhexanoate, stearyl 2-ethylhexanoate, propylene glycol dicaprate, propylene glycol dioleate, isononyl isononanoate, diisopropyl sebacate, propylene glycol isostearate, 2-ethylhexyl paramethoxycinnamate, 2-ethoxyethyl paramethoxycinnamate, isopropyl paramethoxycinnamate/diisopropyl cinnamate ester mixture, methyl bis(trimethylsiloxy) silyl isopentyl trimethoxycinnamate, amyl paradimethylaminobenzoate, 2-ethylhexyl paradimethylaminobenzoate, ethylene glycol salicylate, 2-ethylhexyl salicylate, benzyl salicylate, homomenthyl salicylate, octocrylene, and dimethyl diethyl benzalmalonate; and branched or unsaturated higher alcohols such as 2-octyldodecanol, isostearyl alcohol, and oleyl alcohol. The content of the nonvolatile liquid oil agent is preferably less than 50% by mass, more preferably less than 40% by mass, still more preferably less than 30% by mass, even more preferably less than 20% by mass, and even more preferably less than 10% by mass in the composition.

The composition preferably has a low content of volatile cyclic silicone such as decamethylcyclopentasiloxane from the viewpoint of the drying rate after application. This is because the volatile cyclic silicone has a slower volatilization time than other volatile oils and tends to take a longer time to dry after the application to the keratin substance or the fiber for a head decoration product. The content of the volatile cyclic silicone in the composition is preferably less than 5% by mass, more preferably less than 2% by mass, still more preferably less than 1% by mass, even more preferably less than 0.5% by mass, even more preferably less than 0.1% by mass, and even more preferably 0% by mass in the composition.

The composition preferably has a low content of polyhydric alcohol from the viewpoint of improving color durability and the effect of preventing adhesion of dirt. Examples of the polyhydric alcohol include polyhydric alcohols having a boiling point of more than 260°C at normal pressure, and propylene glycol, and glycerol can be exemplified. The content of the polyhydric alcohol is preferably less than 5% by mass, more preferably less than 2% by mass, still more preferably less than 1% by mass, even more preferably less than 0.5% by mass, and even more preferably less than 0.1% by mass in the composition.

(Formulation)

The formulation of the composition is not particularly limited, and can be made into a formulation such as a liquid, paste, cream, gel, foam, spray, and wax according to the product form. In addition, the composition is preferably a non-aqueous composition. Here, the non-aqueous composition means a composition having a water content of less than 1% by mass, preferably less than 0.5% by mass, and more preferably less than 0.1% by mass.

The formulation of the composition may be an aerosol type. Examples of the aerosol type preparation include a preparation in which the composition, which is an aerosol stock solution, and a propellant are filled in an aerosol container. The constitution of the composition as an aerosol stock solution and the preferred range thereof are the same as described above.

Examples of the propellant used in the aerosol type preparation include liquefied petroleum gas (LPG) which is ethane, propane, normal butane, isobutane, isopentane and a mixture thereof; ethers such as dimethyl ether; and compressed gases such as nitrogen and carbon dioxide, and one or more of these can be used.

In the aerosol preparation, the amount ratio of the composition, which is an aerosol stock solution, to the propellant varies depending on the type of the propellant, but from the viewpoint of aerosol performance, the mass ratio of the composition to the propellant is preferably in the range of 1:0.01 to 1:10, and more preferably in the range of 1:0.05 to 1:7.5.

When the propellant is LPG, the mass ratio of the composition to LPG is still more preferably 1:0.5 to 1:5; when the propellant is dimethyl ether, the mass ratio of the composition to dimethyl ether is still more preferably 1:0.1 to 1:5; and when the propellant is carbon dioxide, the mass ratio of the composition to carbon dioxide is still more preferably 1:0.1 to 1:0.5.

Examples of the aerosol container used for the aerosol type preparation include a known pressure-resistant container made of metal or plastic, and a double-structure container in which an inner bag is accommodated inside the pressure-resistant container. In the double-structure container, it is preferable that the inner bag is filled with the aerosol stock solution, and a propellant is filled between the pressure-resistant container and the inner bag.

As the type of the composition, a cosmetic composition is preferable, and examples thereof include a skin cosmetic composition, a cosmetic composition for eyebrows or eyelashes, a hair cosmetic composition, and a nail cosmetic composition. The hair cosmetic composition can also be applied to fibers for head decoration products.

Examples of the skin cosmetic composition include various skin cosmetic compositions for make-up and sunscreen.

Examples of the cosmetic composition for eyebrows or eyelashes include mascara top coat and eyebrow mascara top coat.

Examples of the hair cosmetic composition include a conditioning agent composition, a treatment agent composition (including a leave-on type), and a styling agent composition.

Examples of the nail cosmetic composition include a manicure and a nail top coat.

Among the above, from the viewpoint of forming a film, the cosmetic composition is preferably a hair cosmetic composition, and more preferably a conditioning agent composition, a treatment agent composition, or a styling agent composition.

In addition, from the viewpoint of forming a film, the composition is preferably a socalled leave-on preparation which is used without being washed off after being applied to keratin substances or fibers for head decoration products by application.

<Application Method of Composition>

The keratin substance or the fiber for a head decoration product when the composition is applied may be in a dry state or a wet state, but from the viewpoint of obtaining the effect of the present invention, it is preferable to apply the composition to the keratin substance or the fiber for a head decoration product in a dry state. The application method of the composition can be appropriately selected depending on the formulation, and examples thereof include coating, casting, and spraying.

From the viewpoint of uniform coatability, it is preferable that the composition is applied to the surface of the keratin substance or the fiber for a head decoration product after being temporarily compatibilized or dispersed before being applied to the keratin substance or the fiber for a head decoration product. As a means for temporarily compatibilization or dispersion, a thermodynamic means such as heating, a physical means such as mechanically applying shear stress, or a chemical means such as adding a compatible solvent can be arbitrarily used. From the viewpoint of user convenience, it is preferable to uniformly compatibilize or disperse the first agent by physical means such as stirring and shaking.

In the method of the present invention, the amount of the composition applied to the keratin substance or the fiber for a head decoration product is not particularly limited, but when applied to the skin or the nail, it is usually in the range of 0.1 mg or more and 1000 mg or less per 1 $cm^2$ of the skin or the nail. When the composition is applied to eyebrows, eyelashes, hair, or fibers for head decoration products, the amount of the composition applied is usually in the range of 0.005 g or more and 1 g or less per 1 g of eyebrows, eyelashes, hair, or fibers for head decoration products.

<Drying Step>

In the method of the present invention, from the viewpoint of forming a film by the composition after applying the composition to the artificially colored or decolored keratin substance or fiber for a head decoration product, it is preferable to air-dry. It is also preferable not to wash away the composition after air-drying. The drying time is not particularly limited as long as the film is substantially formed on the surface of the keratin substance or the fiber for a head decoration product after the application of the composition, and is appropriately adjusted according to the application amount and the application area, but is preferably 4 minutes or less, and more preferably 2 minutes or less.

In addition, when the application object of the present invention is hair or a fiber for a head decoration product,

from the viewpoint of rapidly forming a film, a step of applying the composition to the hair or the fiber for a head decoration product and then drying the hair using a device such as a hood, a hair dryer, or a straightening iron may be performed. From the viewpoint of sustaining the treatment effect, the composition is preferably applied to the hair or the fiber for a head decoration product in a dry state, and then dried, and preferably not washed off.

In a case where the device is used, from the viewpoint of suppressing heat damage to the hair or the fiber for a head decoration product, it is preferable that drying is performed by applying a temperature of 40°C or higher and 220°C or lower. Drying is more preferably performed by a hood or a hair dryer, and the drying temperature is preferably 40°C or higher and 110°C or lower, and more preferably 50°C or higher and 90°C or lower.

The drying time by the device is not particularly limited as long as the film is substantially formed on the surface of the hair or the fiber for a head decoration product, and is appropriately adjusted depending on the amount and quality of the hair or the fiber for a head decoration product, but can be performed in the range of, for example, 10 seconds to 120 minutes.

After drying, brushing may be performed in order to loosen the hair or the fiber for a head decoration product.

[Kit for Treating Keratin Substance or Fiber for Head Decoration Product]

The present invention provides a kit for treating a keratin substance or a fiber for a head decoration product, including a first agent containing one or more selected from the group consisting of a colorant and a decolorant, and a second agent containing the following component (A) and component (B), in which a proportion of a content mass of the component (A) to a total content mass of the component (A) and the component (B) [(A)/{ (A) + (B)}] is 10% or more:

(A) a silicone film-forming agent;
(B) an organopolysiloxane having a degree of polymerization of 100 or more.

The kit of the present invention is preferably a cosmetic kit, more preferably a cosmetic kit for treatment of skin, eyebrows, eyelashes, hair, nails, or fibers for head decoration products, and still more preferably a cosmetic kit for treatment of hair or fibers for head decoration products.

<First Agent>

The first agent is a composition for treating a keratin substance or a fiber for a head decoration product for artificially coloring or decoloring the keratin substance or the fiber for a head decoration product, and contains one or more selected from the group consisting of a colorant and a decolorant.

(Colorant)

Examples of the colorant used in the first agent include pigments and dyes, and the colorant is preferably one or more selected from the group consisting of pigments, direct dyes, and oxidation dyes from the viewpoint of improving color durability, more preferably one or more selected from the group consisting of pigments and direct dyes, and still more preferably pigments from the viewpoint of improving color durability, ease of treatment during coloring treatment, and less damage due to coloring treatment.

The colorant and details thereof are the same as the colorant described in "Artificially Colored Keratin Substance or Fiber for Head Decoration Product".

When the first agent contains a colorant, the content of the colorant in the first agent is preferably 0.01% by mass or more, more preferably 0.1% by mass or more, still more preferably 0.2% by mass or more, and even more preferably 0.3% by mass or more, from the viewpoint of imparting desired colorability, and is preferably 50% by mass or less, and more preferably 30% by mass or less, from the viewpoint of dispersibility in the first agent and economic efficiency, and from the viewpoint of maintaining a good feel. Thus, the content of the colorant in the first agent is preferably 0.01% by mass or more and 50% by mass or less, more preferably 0.1% by mass or more and 50% by mass or less, still more preferably 0.2% by mass or more and 30% by mass or less, and even more preferably 0.3% by mass or more and 30% by mass or less.

(Decolorant)

Examples of the decolorant used in the first agent include an oxidizing agent such as hydrogen peroxide. When the first agent contains a decolorant, the content of the decolorant in the first agent is preferably 0.01% by mass or more, more preferably 0.1% by mass or more, still more preferably 0.2% by mass or more, and even more preferably 0.3% by mass or more, from the viewpoint of imparting desired bleachability, and is preferably 50% by mass or less, and more

preferably 30% by mass or less, from the viewpoint of suppressing deterioration of the keratin substance or the fiber for a head decoration product. Thus, the content of the decolorant in the first agent is preferably 0.01% by mass or more and 50% by mass or less, more preferably 0.1% by mass or more and 50% by mass or less, still more preferably 0.2% by mass or more and 30% by mass or less, and even more preferably 0.3% by mass or more and 30% by mass or less.

In addition to the colorant and the decolorant, the first agent can contain components usually used in a cosmetic composition, for example, a polymer, a functional powder other than the colorant, a solvent, a nonvolatile oil agent, an antioxidant, a perfume, a pigment, an antiseptic, a viscosity improver, a pH modifier, a blood circulation promoting agent, a cooling agent, an antiperspirant, a bactericide, a skin activating agent, a moisturizing agent, and a refreshing agent.

As the type of the first agent containing the colorant, a cosmetic composition is preferable, and examples thereof include a skin cosmetic composition, a cosmetic composition for eyebrows or eyelashes, a hair cosmetic composition, and a nail cosmetic composition.

Examples of the skin cosmetic composition include various skin cosmetic compositions containing a colorant for make-up or foundation.

Examples of the cosmetic composition for eyebrows or eyelashes include various cosmetic compositions for eyebrows or eyelashes containing a colorant, such as mascara, mascara base, and eyebrow mascara.

Examples of the hair cosmetic composition include a hair dye composition. The hair cosmetic composition can also be applied to fibers for head decoration products.

Examples of the nail cosmetic composition include a nail cosmetic composition containing a colorant, such as a manicure and a base coat for a nail.

Examples of the type of the first agent containing a decolorant include a bleaching agent for hair.

Among the above, the first agent is preferably a cosmetic composition containing a colorant, more preferably a hair cosmetic composition, and still more preferably a hair dye composition.

The first agent may be composed of two or more compositions. These compositions are mixed before use to prepare the first agent, which can be applied to a keratin substance or a fiber for a head decoration product.

<Second Agent>

The second agent is a composition for treating a keratin substance or a fiber for a head decoration product, which contains the component (A) and the component (B), in which a proportion of a content mass of the component (A) to a total content mass of the component (A) and the component (B) [(A)/ f (A) + (B)}] is 10% or more. The second agent and preferred embodiments thereof are the same as those of the composition of the present invention.

The second agent may also be composed of two or more compositions. These compositions are mixed before use to prepare the second agent, which can be applied to a keratin substance or a fiber for a head decoration product.

The kit of the present invention may be provided with at least the first agent and the second agent, and may further be provided with a preparation that does not correspond to either the first agent or the second agent.

The method of using the kit of the present invention is not particularly limited as long as the first agent and the second agent are sequentially applied to the keratin substance or the fiber for a head decoration product. Typically, first, the first agent is applied to the keratin substance or the fiber for a head decoration product to artificially perform a coloring treatment or a decoloring treatment, and then the second agent is applied. The application method of the first agent and the second agent can be appropriately selected according to the type of the keratin substance or the fiber for a head decoration product to be applied, and the formulation of the first agent and the second agent. The application method of the second agent and preferred embodiments thereof are the same as those of the composition used in the method of the present invention.

Regarding the above-mentioned embodiments, the present invention discloses the following.

<1> A method for treating an artificially colored or decolored keratin substance or fiber for a head decoration product with a composition, the method including a step of applying the composition to the keratin substance or the fiber for a head decoration product, in which the composition contains the following component (A) and component (B):

(A) a silicone film-forming agent;
(B) an organopolysiloxane having a degree of polymerization of 100 or more, and

whrein a proportion of a content mass of the component (A) to a total content mass of the component (A) and the component (B) [(A)/ f (A) + (B)}] is 10% or more.

<2> The method according to <1>, in which the keratin substance is preferably one or more selected from the group consisting of skin, eyebrows, eyelashes, hair, and nails, and more preferably hair.

<3> The method according to <1> or <2>, in which the head decoration product is one or more selected from the group consisting of a hair wig, a wig, weaving, hair extension, a blade hair, a hair accessory, and a doll hair.

<4> The method according to any one of <1> to <3>, in which the keratin substance or the fiber for a head decoration product is preferably one or more selected from the group consisting of skin, eyebrows, eyelashes, hair, nails, and

fibers for head decoration products, more preferably one or more selected from the group consisting of hair and fibers for head decoration products, and still more preferably hair.

<5> The method according to any one of <1> to <4>, in which the artificially colored keratin substance or fiber for a head decoration product is a keratin substance or fiber for a head decoration product which has been subjected to a coloring treatment using a colorant, preferably one or more selected from the group consisting of a pigment, a direct dye, and an oxidation dye, more preferably one or more selected from the group consisting of a pigment and a direct dye, and still more preferably a pigment.

<6> The method according to any one of <1> to <4>, in which the artificially decolored keratin substance or fiber for a head decoration product is a keratin substance or fiber for a head decoration product which has been artificially bleached with a bleaching agent.

<7> The method according to any one of <1> to <6>, in which the method is a method of treating, preferably artificially colored or decolored skin, eyebrows, eyelashes, hair, nails, or fibers for head decoration products, more preferably artificially colored or decolored hair or fibers for head decoration products, still more preferably artificially colored or decolored hair, even more preferably artificially colored hair, and even more preferably hair dyed with a hair dye, with the composition.

<8> The method according to <7>, in which the hair dye is a hair dye containing preferably a colorant, more preferably one or more selected from the group consisting of a pigment, a direct dye, and an oxidation dye, still more preferably one or more selected from the group consisting of a pigment and a direct dye, and even more preferably a pigment.

<9> The method according to any one of <1> to <8>, in which the component (A) is one or more selected from the group consisting of the following components (A1) and (A2): component (A1): a silicone resin represented by an average formula $(R^1)_mSiO_{(4-m)/2}$ (in which, $R^1$ represents a hydrocarbon group having 1 or more and 12 or less carbon atoms which may be fluorine-substituted or a hydroxy group; a plurality of $R^1$'s may be the same as or different from each other; and m is an average number which is more than 0 and less than 3), the silicone resin containing one or more units selected from the group consisting of a T unit represented by $R^1SiO_{3/2}$ and a Q unit represented by $SiO_{4/2}$; and

component (A2): a silicone polymer containing a polysiloxane moiety and a moiety formed of a non-silicone organic chain.

<10> The method according to <9>, in which the component (A1) includes one or more selected from the group consisting of the following component (A1-1) and component (A1-2): (A1-1) a silicone resin represented by the above average formula, containing a T unit represented by $R^1SiO_{3/2}$ and substantially not containing a Q unit represented by $SiO_{4/2}$, and (A1-2) a silicone resin represented by the above average formula, and containing a Q unit represented by $SiO_{4/2}$ and an M unit represented by $(R1)_3SiO_{1/2}$.

<11> The method according to <9> or <10>, in which the component (A2) preferably includes one or more selected from the group consisting of the following components (A2-1) to (A2-4), and more preferably includes a component (A2-1):

(A2-1) An acryl silicone polymer;
(A2-2) A silicone-modified alicyclic structure-containing polymer;
(A2-3) A silicone-modified pullulan;
(A2-4) A polyurea/urethane silicone.

<12> The method according to <10> or <11>, in which the component (A1-1) is preferably polysilsesquioxanes, more preferably one or more selected from the group consisting of polymethylsilsesquioxane, polypropylsilsesquioxane, polyphenylsilsesquioxane, polymethylphenylsilsesquioxane, and fluorine-modified alkyldimethylpolysilsesquioxane, still more preferably one or more selected from the group consisting of polymethylsilsesquioxane and polypropylsilsesquioxane, and even more preferably polymethylsilsesquioxane.

<13> The method according to <10> or <11>, in which the component (A1-2) is preferably one or more selected from the group consisting of trimethylsiloxysilicate, trifluoropropyldimethyltrimethylsiloxysilicate and (trimethylsiloxysilicate/dimethiconol) crosspolymer, more preferably one or more selected from the group consisting of trimethylsiloxysilicate, and trifluoropropyldimethyltrimethylsiloxysilicate, and still more preferably trimethylsiloxysilicate.

<14> The method according to any one of <11> to <13>, in which the acryl silicone polymer of the component (A2-1) is one or more selected from the group consisting of an acrylic polymer having a carbosiloxane dendrimer structure in the side chain, an acryl-silicone graft copolymer, and a graft-type copolymer or alternate block-type copolymer where a structural unit of a polysiloxane group and a structural unit of a polymer of an unsaturated monomer via a sulfide bond, preferably one or more selected from the group consisting of an acrylic polymer having a carbosiloxane dendrimer structure in the side chain and an acryl-silicone graft copolymer, more preferably one or more selected from the group consisting of (acrylates/polytrimethylsiloxymethacrylate) copolymer and (acrylates/dimethicone) copolymer, and still more preferably (acrylates/dimethicone) copolymer.

<15> The method according to any one of <11> to <14>, in which the component (A) preferably includes one or more selected from the group consisting of the component (A1), the component (A2-1), and the component (A2-2), more preferably includes one or more selected from the group consisting of the component (A1) and the component (A2-1), still more preferably includes one or more selected from the group consisting of the component (A1-2) and the component (A2-1), and even more preferably includes the component (A1-2).
<16> The method according to any one of <9> to <15>, in which the component (A) contains both the component (A1) and the component (A2).
<17> The method according to <16>, in which the component (A) includes the component (A1) and one or more selected from the group consisting of the component (A2-1) and the component (A2-2), and preferably includes the component (A1-2) and the component (A2-1).
<18> The method according to any one of <1> to <17>, in which the component (A) contains one or more selected from the group consisting of trimethylsiloxysilicate, phenylpropyldimethylsiloxysilicate, trifluoropropyldimethyltrimethylsiloxysilicate, (trimethylsiloxysilicate/dimethiconol) crosspolymer, polymethylsilsesquioxane, polypropylsilsesquioxane, (acrylates/polytrimethylsiloxymethacrylate) copolymer, (acrylates/dimethicone) copolymer, and (norbornene/tris(trimethylsiloxy)silylnorbomene) copolymer, preferably contains one or more selected from the group consisting of trimethylsiloxysilicate, phenylpropyldimethylsiloxysilicate, trifluoropropyldimethyltrimethylsiloxysilicate, (trimethylsiloxysilicate/dimethiconol) crosspolymer, polymethylsilsesquioxane, polypropylsilsesquioxane, (acrylates/polytrimethylsiloxymethacrylate) copolymer, and (acrylates/dimethicone) copolymer, more preferably contains one or more selected from the group consisting of trimethylsiloxysilicate, polymethylsilsesquioxane, and (acrylates/dimethicone) copolymer, still more preferably contains trimethylsiloxysilicate, and even more preferably is trimethylsiloxysilicate.
<19> The method according to any one of <1> to <18>, in which the degree of polymerization of the component (B) is preferably 100 or more and 4,300 or less, more preferably 150 or more and 4,200 or less, still more preferably 250 or more and 4,200 or less, even more preferably 300 or more and 4,000 or less, even more preferably 320 or more and 3,800 or less, even more preferably 320 or more and 3,700 or less, and even more preferably 350 or more and 3,650 or less.
<20> The method according to any one of <1> to <19>, in which the component (B) is an organopolysiloxane represented by the following general formula (1):

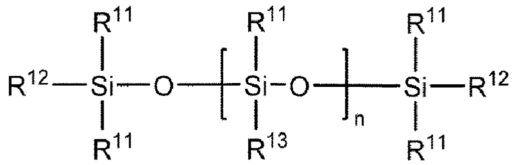
(1)

in which each $R^{11}$ independently represents a hydrocarbon group having 1 or more and 6 or less carbon atoms, each $R^{12}$ independently represents a hydroxy group, an alkoxy group having 1 or more and 6 or less carbon atoms, or a hydrocarbon group having 1 or more and 6 or less carbon atoms, $R^{13}$ represents a hydrocarbon group having 1 or more and 6 or less carbon atoms or a primary to tertiary amino group-containing group, n represents a degree of polymerization and is a number of 100 or more, and n's $R^{13}$s may be the same or different from each other.
<21> The method according to any one of <1> to <20>, in which the viscosity at 25°C of the component (B) is preferably 120 $mm^2/s$ or more and 100,000,000 $mm^2/s$ or less, more preferably 200 $mm^2/s$ or more and 80,000,000 $mm^2/s$ or less, still more preferably 500 $mm^2/s$ or more and 80,000,000 $mm^2/s$ or less, even more preferably 700 $mm^2/s$ or more and 80,000,000 $mm^2/s$ or less, even more preferably 700 $mm^2/s$ or more and 50,000,000 $mm^2/s$ or less, even more preferably 800 $mm^2/s$ or more and 40,000,000 $mm^2/s$ or less, even more preferably 800 $mm^2/s$ or more and 35,000,000 $mm^2/s$ or less, even more preferably 800 $mm^2/s$ or more and 30,000,000 $mm^2/s$ or less, and even more preferably 1,000 $mm^2/s$ or more and 25,000,000 $mm^2/s$ or less.
<22> The method according to any one of <1> to <21>, in which the component (B) is one or more selected from the group consisting of dimethylpolysiloxane, methylphenylpolysiloxane, aminopropylmethylpolysiloxane, and dimethiconol, and more preferably dimethylpolysiloxane.
<23> The method according to any one of <1> to <22>, in which the content of the component (A) in the composition is preferably 0.1% by mass or more and 30% by mass or less, more preferably 0.5% by mass or more and 25% by mass or less, still more preferably 1% by mass or more and 20% by mass or less, even more preferably 2% by mass or more and 15% by mass or less, and even more preferably 3% by mass or more and 15% by mass or less.
<24> The method according to any one of <1> to <23>, in which the content of the component (B) in the composition is preferably 0.1% by mass or more and 30% by mass or less, more preferably 0.3% by mass or more and 25% by mass or less, still more preferably 0.5% by mass or more and 20% by mass or less, even more preferably 0.5% by

mass or more and 15% by mass or less, and even more preferably 0.5% by mass or more and 10% by mass or less.

<25> The method according to any one of <1> to <24>, in which the total content of the component (A) and the component (B) in the composition is preferably 0.2% by mass or more and 50% by mass or less, more preferably 0.5% by mass or more and 45% by mass or less, still more preferably 1% by mass or more and 40% by mass or less, even more preferably 1% by mass or more and 35% by mass or less, even more preferably 2% by mass or more and 35% by mass or less, even more preferably 2% by mass or more and 25% by mass or less, even more preferably 3% by mass or more and 25% by mass or less, even more preferably 3.5% by mass or more and 25 by mass or less, and even more preferably 5% by mass or more and 20% by mass or less.

<26> The method according to any one of <1> to <25>, in which the proportion of the content mass of the component (A) to the total content mass of the component (A) and the component (B) [(A)/ f (A) + (B)}] in the composition is preferably 10% or more and 98% or less, more preferably 10% or more and 95% or less, still more preferably 20% or more and 95% or less, even more preferably 30% or more and 95% or less, even more preferably 40% or more and 95% or less, and even more preferably 50% or more and 95% or less.

<27> The method according to any one of <1> to <26>, in which the composition further contains, as a component (C), an organopolysiloxane other than the component (A) and the component (B), which has a polyalkylene oxide moiety and a cationic group.

<28> The method according to <27>, in which the cationic group contained in the component (C) is a cationic group or a group which can be ionized to become a cationic group, preferably one or more selected from the group consisting of a primary amino group, a secondary amino group, a tertiary amino group, and a quaternary ammonium group, and more preferably one or more selected from the group consisting of a primary amino group, a secondary amino group, and a tertiary amino group.

<29> The method according to <27> or <28>, in which the alkylene oxide constituting the polyalkylene oxide moiety is preferably one or more selected from the group consisting of ethylene oxide, propylene oxide, trimethylene oxide, butylene oxide, and tetramethylene oxide, more preferably one or more selected from the group consisting of ethylene oxide, propylene oxide, and trimethylene oxide, still more preferably one or more selected from the group consisting of ethylene oxide and propylene oxide, and even more preferably ethylene oxide.

<30> The method according to any one of <27> to <29>, in which the average addition mole number of alkylene oxide in the polyalkylene oxide moiety is preferably 2 or more, more preferably 4 or more, and still more preferably 10 or more, and from the viewpoint of availability, is preferably 100 or less, more preferably 80 or less, and still more preferably 50 or less.

<31> The method according to any one of <27> to <30>, in which the component (C) is one or more selected from the group consisting of an aminopolyether-modified silicone (C1) having a repeating unit represented by the general formula (2) shown below and an aminopolyether-modified silicone (C2) having a repeating unit represented by the general formula (3) shown below:

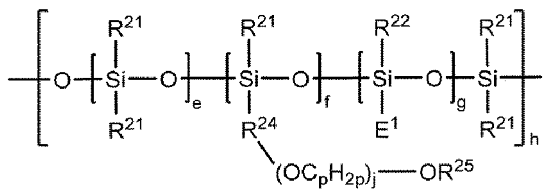

(2)

In the formula (2), $R^{21}$ represents a monovalent hydrocarbon group having 1 or more and 6 or less carbon atoms, $R^{22}$ represents any of $R^{21}$ or $E^1$, $E^1$ represents a monovalent group represented by $-R^{23}-Z^1$ (where $R^{23}$ represents a divalent hydrocarbon group having 1 or more and 6 or less carbon atoms, and $Z^1$ represents a primary to tertiary amino group-containing group), $R^{24}$ represents a divalent hydrocarbon group having 1 or more and 6 or less carbon atoms, $R^{25}$ represents a hydrogen atom or a monovalent hydrocarbon group having 1 or more and 4 or less carbon atoms.

e is a number of 1 or more and 50 or less, f is a number of 1 or more and 50 or less, g is a number of 1 or more and 50 or less, h is a number of 1 or more, j is a number of 2 or more and 100 or less, p is a number of 2 or more and 10 or less, the bonding order of the parenthesized structural units is not limited, and the bonding form thereof may be in a block form or a random form, j's $(OC_pH_{2p})$s can be the same or different, and plural $R^{21}$'s, $R^{22}$'s, $R^{24}$'s, $R^{25}$'s and $E^1$'s can be the same or different.

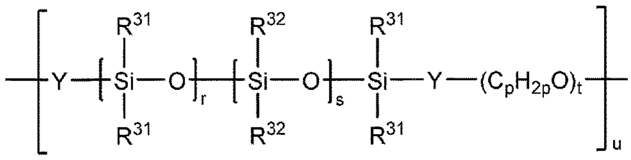

(3)

In the formula (3), $R^{31}$ represents a monovalent hydrocarbon group having 1 or more and 6 or less carbon atoms, $R^{32}$ represents any of $R^{31}$ or $E^2$, $E^2$ represents a monovalent group represented by $-R^{33}-Z^2$ (where $R^{33}$ represents a single bond, or a divalent hydrocarbon group having 1 or more and 20 or less carbon atoms, and $Z^2$ represents a primary to tertiary amino group-containing group), Y represents a single bond, or a divalent group having 1 or more and 12 or less carbon atoms. In the case where all $R^{32}$'s are $R^{31}$'s, at least one Y is an amino group-containing divalent group. In the case where all Y's are divalent groups not containing an amino group, at least one $R^{32}$ is $E^2$. r is a number of 1 or more, s is a number of 1 or more, t is a number of 2 or more and 100 or less, and u is a number of 1 or more. p is the same as above, and is a number of 2 or more and 10 or less. The bonding order of the parenthesized structural units is not limited, and the bonding form thereof may be in a block form or a random form, t's $(C_pH_{2p}O)$s can be the same or different. Plural $R^{31}$'s, $R^{32}$'s, $E^2$'s and Y's can be the same or different.

<32> The method according to <31>, in which the component (C1) is represented by the following general formula (2-1):

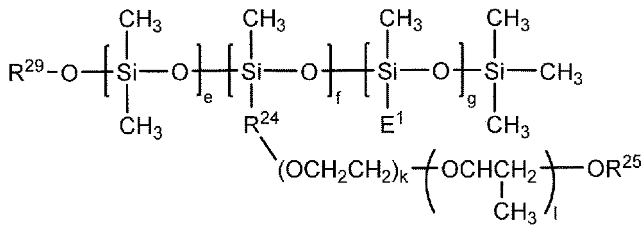

(2-1)

In the formula (2-1), $E^1$, $R^{24}$, $R^{25}$, e, f, and g are the same as above, $R^{29}$ represents a monovalent hydrocarbon group having 1 or more and 4 or less carbon atoms, or a trimethylsilyl group, k is a number of 1 or more and 50 or less, l is a number of 0 or more and 50 or less, and is preferably a number of 1 or more and 50 or less.

<33> The method according to <31> or <32>, in which the component (C2) is one or more selected from the group consisting of an aminopolyether-modified silicone having a structure represented by the following general formula (3-1) and an aminopolyether-modified silicone having a structure represented by the following general formula (3-2):

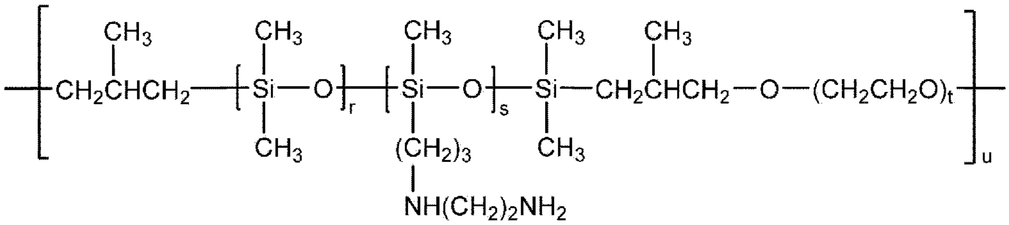

(3-1)

In the formula (3-1), r, s, t and u are the same as above.

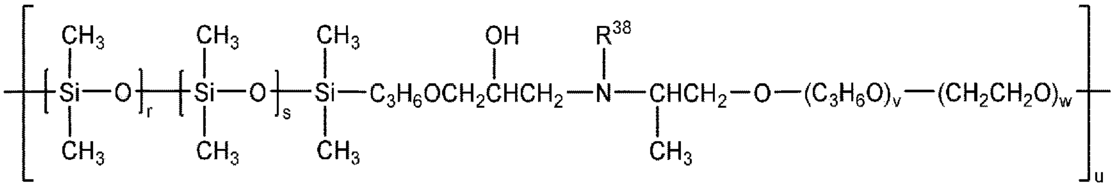

(3-2)

In the formula (3-2), $R^{38}$, r, s and u are the same as above, v is a number of 0 or more and 50 or less, and preferably a number of 2 or more and 50 or less. w is a number of 2 or more and 100 or less. v + w is a number of 2 or more and 100 or less, preferably a number of 4 or more and 80 or less, and more preferably a number of 10 or more and

50 or less.

<34> The method according to any one of <31> to <33>, in which the component (C) is preferably an aminopolyether-modified silicone (C2) having a repeating unit represented by the general formula (3), more preferably one or more selected from the group consisting of an aminopolyether-modified silicone having a structure represented by the general formula (3-1) and an aminopolyether-modified silicone having a structure represented by the general formula (3-2), still more preferably an aminopolyether-modified silicone having a structure represented by the general formula (3-1), and even more preferably an aminopolyether-modified silicone composed of a structure represented by the general formula (3-1).

<35> The method according to any one of <27> to <34>, in which the content of the component (C) in the composition is preferably 0.01% by mass or more and 25% by mass or less, more preferably 0.1% by mass or more and 15% by mass or less, still more preferably 0.5% by mass or more and 12% by mass or less, even more preferably 0.6% by mass or more and 10% by mass or less, and even more preferably 0.7% by mass or more and 5% by mass or less.

<36> The method according to any one of <1> to <35>, in which the composition further contains a solvent as a component (D).

<37> The method according to <36>, in which the component (D) contains one or more solvents selected from the group consisting of dimethylpolysiloxane having a viscosity at 25°C of 4 $mm^2/s$ or less, methyltrimethicone, pentane, isopentane, hexane, isohexane, heptane, isoheptane, decane, isodecane, dodecane, isododecane, tridecane, isotridecane, tetradecane, isotetradecane, and light liquid isoparaffin, preferably one or more solvents selected from the group consisting of dimethylpolysiloxane having a viscosity at 25°C of 4 $mm^2/s$ or less, methyltrimethicone, isodecane, isododecane, isotetradecane, and light liquid isoparaffin, and more preferably one or more solvents selected from the group consisting of isodecane, isododecane, isotetradecane, and light liquid isoparaffin.

<38> The method according to <36> or <37>, in which the content of the component (D) in the composition is preferably 40% by mass or more and 99% by mass or less, more preferably 50% by mass or more and 99% by mass or less, and still more preferably 60% by mass or more and 98% by mass or less.

<39> The method according to any one of <1> to <38>, in which the content of water in the composition is preferably 10% by mass or less, more preferably less than 5% by mass, and still more preferably less than 2% by mass.

<40> The method according to any one of <1> to <39>, in which the content of polysilicone-polyamide copolymer in the composition is preferably less than 5% by mass, more preferably less than 2% by mass, still more preferably less than 1% by mass, even more preferably less than 0.5% by mass, even more preferably less than 0.1% by mass, and even more preferably 0% by mass.

<41> The method according to any one of <1> to <40>, in which the composition is a cosmetic composition, preferably a hair cosmetic composition, more preferably a conditioning agent composition, a treatment agent composition, or a styling agent composition.

<42> The method according to any one of <1> to <41>, in which the composition is a leave-on preparation.

<43> The method according to any one of <1> to <42>, in which the composition is applied to a keratin substance or a fiber for a head decoration product in a dry state.

<44> A kit for treating a keratin substance or a fiber for a head decoration product, including a first agent containing one or more selected from the group consisting of a colorant and a decolorant, and a second agent containing the following component (A) and component (B), in which a proportion of a content mass of the component (A) to a total content mass of the component (A) and the component (B) [(A)/{(A) + (B)}] is 10% or more:

(A) a silicone film-forming agent;
(B) an organopolysiloxane having a degree of polymerization of 100 or more.

<45> The kit according to <44>, in which the colorant in the first agent is a pigment or a dye, preferably one or more selected from the group consisting of a pigment, a direct dye, and an oxidation dye, more preferably one or more selected from the group consisting of a pigment and a direct dye, and still more preferably a pigment.

<46> The kit according to <44> of <45>, in which the content of the colorant in the first agent is preferably 0.01% by mass or more and 50% by mass or less, more preferably 0.1% by mass or more and 50% by mass or less, still more preferably 0.2% by mass or more and 30% by mass or less, and even more preferably 0.3% by mass or more and 30% by mass or less.

<47> The kit according to any one of <44> to <46>, in which the first agent is preferably a cosmetic composition containing a colorant, more preferably a hair cosmetic composition, and still more preferably a hair dye composition.

<48> The kit according to any one of <44> to <47>, in which the total content of the component (A) and the component (B) in the second agent is preferably 0.2% by mass or more and 50% by mass or less, more preferably 0.5% by mass or more and 45% by mass or less, still more preferably 1% by mass or more and 40% by mass or less, even more preferably 2% by mass or more and 35% by mass or less, even more preferably 3% by mass or more and 25% by mass or less, even more preferably 5% by mass or more and 20% by mass or less, and even more preferably

5% by mass or more and 15% by mass or less.

<49> The kit according to any one of <44> to <48>, in which the proportion of the content mass of the component (A) to the total content mass of the component (A) and the component (B) [(A)/{(A) + (B)}] in the second agent is preferably 10% or more and 98% or less, more preferably 10% or more and 95% or less, still more preferably 20% or more and 95% or less, even more preferably 30% or more and 95% or less, even more preferably 40% or more and 95% or less, and even more preferably 50% or more and 95% or less.

Examples

Hereinafter, the present invention will be described with reference to Examples, but the present invention is not limited to the scope of Examples. In these Examples, various measurements and evaluations were performed by the following methods.

<Hair Treatment Method>

A bundle of human gray hair (100%) (manufactured by Beaulax Corporation, length: 10 cm, mass: 1 g) was washed with a plain shampoo having the following composition, rinsed with warm water at 40°C, and sufficiently dried. To this hair bundle, 0.30 g of a hair dye composition (isododecane dispersion of carbon black (1% by mass)) having the following composition was applied, and then dried with a dryer (artificial coloring). Further, 0.20 g of the hair cosmetic composition of each example was applied, and then dried with a dryer to perform hair treatment.

(Composition of Plain Shampoo)

Component (% by mass)
Sodium polyoxyethylene(2) lauryl ether sulfate (*1) 15.5
Lauric acid diethanolamide (*2) 1.5
Edetate tetrasodium salt 0.3
Sodium benzoate 1.43
Purified water balance
Total 100.0
*1: 57.4% by mass as EMAL 227 (manufactured by Kao Corporation, active ingredient 27% by mass)
*2: AMINON L-02 (manufactured by Kao Corporation)

(Composition of Hair Dye Composition)

Component (% by mass)
Carbon black (*1) 1.0
Isododecane (*2) balance
Total 100.0
*1: ISD-CB2, manufactured by Daito Kasei Kogyo Co., Ltd., isododecane solution of carbon black (15% by mass)
*2: Marukazol R, manufactured by Maruzen Petrochemical Co., Ltd., isododecane

<Color Durability (Hair Washing Resistance)>

After the treated hair bundle was subjected to colorimetry using a D65 light source of a color difference meter (CR-400, manufactured by Konica Minolta, Inc.) in the CIE color system (L*, a*, b*), the hair bundle was washed with a plain shampoo having the above-described composition using warm water at 40°C, and dried, and this process was performed seven times. The hair bundle after being washed and dried seven times was similarly color-measured by a color difference meter, and the color difference (ΔE*) from the hair bundle before being washed was calculated according to the following equation. The measurement of L*, a*, and b* was performed at six different points on the hair bundle (one point on each of the front and back sides of the central portion of each region obtained by dividing the hair bundle into three equal parts in the length direction), and the average value was calculated.

$$\Delta E^* = \sqrt{(L_1^* - L_0^*)^2 + (a_1^* - a_0^*)^2 + (b_1^* - b_0^*)^2}$$

$L_0$*, $a_0$*, $b_0$*: measured values of hair bundle before hair washing
$L_1$*, $a_1$*, bi*: measured values of hair bundle after seven times of hair washing

When ΔE* < 17, it can be determined that the hair bundle after treatment has good hair washing resistance and excellent color durability. Further, when ΔE* < 15, it is better, when ΔE* < 10, it is further better, and when ΔE* < 5, it is still further better.

<Difficulty in Adhesion of Dirt>

20 μL of the hair cosmetic composition of each example was added dropwise to a commercially available PET film (Lumirror Film T60-A4-100 μm, manufactured by Toray Industries, Inc.), stretched with a bar coater (No. 3, manufactured by AS ONE Corporation), and air-dried to form a coating film, and then the coating film-formed portion was cut out to a size of 3 cm × 3 cm to prepare a test film. The mass of this film is defined as $F_0$ [g].

As a model of physical contamination, zirconia beads ("YTZ balls" manufactured by Nikkato Corporation, φ = 1 mm) were used. 10.4 g of zirconia beads were placed in a balance dish (BD-2, manufactured by AS ONE Corporation) and leveled, and the coating film surface of the film cut into a size of 3 cm × 3 cm was brought into contact therewith. Next, a balance dish was placed on the film, and a weight, which was obtained by adding tap water to a standard bottle (No. 11, manufactured by AS ONE Corporation) and setting the weight to 200 g, was placed on the balance dish for 30 seconds. Then, the weight was removed, and the mass ($F_1$ [g]) of the film to which the beads were attached was measured.

From the change in mass of the film before and after the contact with the beads, the adhesion rate of the beads was calculated by the following formula. As the mass of the beads covering the area of the test film of 3 cm × 3 cm by 100%, 2.68 g (average value of two measurements) was used.

Adhesion rate of beads [%] = ($F_1$ - $F_0$ [g]) ÷ (mass of beads covering the area of the test film by 100% [g]) × 100

The above test was performed twice, and the average value was shown in the table. It can be said that when the adhesion rate is 25% or less, dirt is difficult to adhere, when the adhesion rate is 20% or less, dirt is more difficult to adhere, when the adhesion rate is 15% or less, dirt is still more difficult to adhere, and when the adhesion rate is 5% or less, dirt is even more difficult to adhere.

Examples 1 to 19 and Comparative Examples 1 to 3 (Preparation and Evaluation of Hair Cosmetic Composition)

Each of the components shown in Tables 1 to 4 was blended at the blending ratio described in each table, and mixed until homogeneous to prepare a hair cosmetic composition. Using the obtained hair cosmetic composition, evaluation was performed by the method described above. The results are shown in Tables 1 to 4.

The blending amounts (% by mass) described in the tables are all amounts of active ingredients.

Table 1

| (% by mass) | | | Example | Comparative Example | | Example |
|---|---|---|---|---|---|---|
| | | | 1 | 1 | 2 | 2 |
| (A) | (A1-2) Trimethylsiloxysilicate | X-21-5595 *1 | 8.0 | 8.0 | 0.0 | 1.2 |
| (B) | Dimethylpolysiloxane viscosity 20,000,000 $mm^2/s$ | Silsoft B3020 *2 | 4.0 | 0.0 | 4.0 | 10.8 |
| (D) | Isododecane | Marukazol R *3 | Balance | Balance | Balance | Balance |
| Total | | | 100 | 100 | 100 | 100 |
| (A)+(B) Total content (% by mass) | | | 12.0 | 8.0 | 4.0 | 12.0 |
| (A)/{(A)+(B)} (%) | | | 67 | 100 | 0 | 10 |

(continued)

| (% by mass) | | | Example | Comparative Example | | Example |
|---|---|---|---|---|---|---|
| | | | 1 | 1 | 2 | 2 |
| Evaluation result | Color durability (Hair washing resistance) | ΔE* after 7 hair washes | 5.1 | 7.5 | 23.2 | 15.4 |
| | Difficulty in adhesion of dirt | Adhesion rate (%) of zirconia beads | 4.7 | - | 0.6 | 9.0 |

Table 1 (continued)

| (% by mass) | | | Example | | | | Comparative Example |
|---|---|---|---|---|---|---|---|
| | | | 3 | 4 | 5 | 6 | 3 |
| (A) | (A1-2) Trimethylsiloxysilicate | X-21-5595 *1 | 2.0 | 5.0 | 10.0 | 11.4 | 0.6 |
| (B) | Dimethylpolysiloxane viscosity 20,000,000 $mm^2/s$ | Silsoft B3020 *2 | 10.0 | 7.0 | 2.0 | 0.6 | 11.4 |
| (D) | Isododecane | Marukazol R *3 | Balance | Balance | Balance | Balance | Balance |
| Total | | | 100 | 100 | 100 | 100 | 100 |
| (A)+(B) Total content (% by mass) | | | 12.0 | 12.0 | 12.0 | 12.0 | 12.0 |
| (A)/{(A)+(B)} (%) | | | 17 | 42 | 83 | 95 | 5 |
| Evaluation result | Color durability (Hair washing resistance) | $\Delta E^*$ after 7 hair washes | 14.8 | 6.1 | 5.3 | 4.9 | 18.9 |
| | Difficulty in adhesion of dirt | Adhesion rate (%) of zirconia beads | 7.5 | 13.6 | 0.0 | 0.0 | 6.5 |

Table 2

| (% by mass) | | | Example | | |
|---|---|---|---|---|---|
| | | | 1 | 7 | 8 |
| (A) | (A1-2) Trimethylsiloxysilicate | X-21-5595 *1 | 8.0 | 2.0 | 15.0 |
| | | SR1000 *4 | | | |
| (B) | Dimethylpolysiloxane viscosity 20,000,000 $mm^2/s$ | Silsoft B3020 *2 | 4.0 | 1.0 | 7.5 |
| (D) | Isododecane | Marukazol R *3 | Balance | Balance | Balance |
| Total | | | 100 | 100 | 100 |
| (A)+(B) Total content (% by mass) | | | 12.0 | 3.0 | 22.5 |
| (A)/{(A)+(B)} (%) | | | 67 | 67 | 67 |
| Evaluation result | Color durability (Hair washing resistance) | ΔE* after 7 hair washes | 5.1 | 13.9 | 5.9 |
| | Difficulty in adhesion of dirt | Adhesion rate (%) of zirconia beads | 4.7 | 0.4 | 17.5 |

Table 2 continued

| (% by mass) | | | Example | | |
|---|---|---|---|---|---|
| | | | 9 | 10 | 11 |
| (A) | (A1-2) Trimethylsiloxysilicate | X-21-5595 *1 | 20.0 | 24.0 | |
| | | SR1000 *4 | | | 28.4 |
| (B) | Dimethylpolysiloxane viscosity 20,000,000 $mm^2/s$ | Silsoft B3020 *2 | 10.0 | 12.0 | 14.2 |
| (D) | Isododecane | Marukazol R *3 | Balance | Balance | Balance |
| Total | | | 100 | 100 | 100 |
| (A)+(B) Total content (% by mass) | | | 30.0 | 36.0 | 42.6 |
| (A)/{(A)+(B)} (%) | | | 67 | 67 | 67 |
| Evaluation result | Color durability (Hair washing resistance) | ΔE* after 7 hair washes | 6.0 | 4.8 | 8.3 |
| | Difficulty in adhesion of dirt | Adhesion rate (%) of zirconia beads | 16.0 | 20.1 | 25.6 |

Table 3

| (% by mass) | | | Example | | | | | | | |
|---|---|---|---|---|---|---|---|---|---|---|
| | | | 1 | 12 | 13 | 14 | 15 | 16 | 17 | 18 |
| (A) | (A1-2) Trimethylsiloxysilicate | X-21-5595 *1 | 8.0 | 8.0 | 8.0 | 8.0 | 8.0 | 8.0 | 8.0 | 8.0 |
| (B) | Dimethylpolysiloxane viscosity 20,000,000 $mm^2/s$ | Silsoft B3020 *2 | 4.0 | | | | | | | |
| | Dimethylpolysiloxane viscosity 30,000,000 $mm^2/s$ | X-25-9074 *5 | | 4.0 | | | | | | |
| | Dimethylpolysiloxane viscosity 3,000,000 $mm^2/s$ | X-21-5686 *6 | | | 4.0 | | | | | |
| | Dimethylpolysiloxane viscosity 500,000 $mm^2/s$ | KF-96H-500,000cs *7 | | | | 4.0 | | | | |
| | Dimethylpolysiloxane viscosity 300,000 $mm^2/s$ | KF-96H-300,000cs *8 | | | | | 4.0 | | | |
| | Dimethylpolysiloxane viscosity 100,000 $mm^2/s$ | KF-96H-100,000cs *9 | | | | | | 4.0 | | |
| | Dimethylpolysiloxane viscosity 10,000 $mm^2/s$ | KF-96H-10,000cs *10 | | | | | | | 4.0 | |
| | Dimethylpolysiloxane viscosity 1,000 $mm^2/s$ | KF-96-1,000cs *11 | | | | | | | | 4.0 |
| (D) | Isododecane | Marukazol R *3 | Balance | Balance | Balance | Balance | Balance | Balance | Balance | Balance |
| Total | | | 100 | 100 | 100 | 100 | 100 | 100 | 100 | 100 |
| (A)+(B) Total content (% by mass) | | | 12.0 | 12.0 | 12.0 | 12.0 | 12.0 | 12.0 | 12.0 | 12.0 |
| (A)/{(A)+(B)} (%) | | | 67 | 67 | 67 | 67 | 67 | 67 | 67 | 67 |
| Evaluation result | Color durability (Hair washing resistance) | ΔE* after 7 hair washes | 5.1 | 7.8 | 6.6 | 5.1 | 4.7 | 5.8 | 2.0 | 2.7 |
| | Difficulty in adhesion of dirt | Adhesion rate (%) of zirconia beads | 4.7 | 13.4 | 2.6 | 1.5 | 2.2 | 2.4 | 2.6 | 1.5 |

Table 4

| (% by mass) | | | Example | |
|---|---|---|---|---|
| | | | 1 | 19 |
| | (A1-2) Tnmethylsiloxysilicate | X-21-5595 *1 | 8.0 | |
| (A) | (A2-1) (Acrylates/dimethicone) copolymer | KP-550 *12 | | 8.0 |
| (B) | Dimethylpolysiloxane viscosity 20,000,000 $mm^2/s$ | Silsoft B3020 *2 | 4.0 | 4.0 |
| (D) | Isododecane | Marukazol R *3 | Balance | Balance |
| Total | | | 100 | 100 |
| (A)+(B) Total content (% by mass) | | | 12.0 | 12.0 |
| (A)/{(A)+(B)} (%) | | | 67 | 67 |
| Evaluation result | Color durability (Hair washing resistance) | ΔE* after 7 hair washes | 5.1 | 6.7 |
| | Difficulty in adhesion of dirt | Adhesion rate (%) of zirconia beads | 4.7 | 3.2 |

Example 20

A bundle of artificial hair having a length of 10 cm, a width of 1.5 cm, and a mass of 1 g was produced using a regenerated collagen fiber "Nextension Crystal XXL 30" (color number: 30 (white), shape: straight) manufactured by Kaneka Corporation as a fiber for a head decoration product.

Artificial coloring and hair treatment were performed in the same manner as in Example 1 except that the above-described artificial hair bundle was used in place of the human gray hair (100%) bundle in Example 1, and the above-described evaluation was performed. The results are shown in Table 5.

Table 5

| (% by mass) | | | Example | |
|---|---|---|---|---|
| | | | 1 | 20 |
| (A) | (A1-2) Trimethylsiloxysilicate | X-21-5595 *1 | 8.0 | 8.0 |
| (B) | Dimethylpolysiloxane viscosity 20,000,000 $mm^2/s$ | Silsoft B3020 *2 | 4.0 | 4.0 |
| (D) | Isododecane | Manikazol R *3 | Balance | Balance |
| Total | | | 100 | 100 |
| (A)+(B) Total content (% by mass) | | | 12.0 | 12.0 |
| (A)/{(A)+(B)} (%) | | | 67 | 67 |
| Evaluation result | Object to be treated | | Human gray hair | Artificial hair |
| | Color durability (Hair washing resistance) | ΔE * after 7 hair washes | 5.1 | 2.7 |
| | Difficulty in adhesion of dirt | Adhesion rate (%) of zirconia beads | 4.7 | 4.7 |

The ingredients shown in the tables are as follows.

*1: X-21-5595, manufactured by Shin-Etsu Chemical Co., Ltd., isododecane solution of trimethylsiloxysilicate (60% by mass)

*2: Silsoft B3020, manufactured by Momentive Performance Materials, Inc., dimethylpolysiloxane, viscosity at 25°C:

20,000,000 $mm^2/s$ (degree of polymerization: 3,546)
*3: Marukazol R, manufactured by Maruzen Petrochemical Co., Ltd., isododecane
*4: SR1000, manufactured by Momentive Performance Materials, Inc., trimethylsiloxysilicate
*5: X-25-9074, manufactured by Shin-Etsu Chemical Co., Ltd., dimethylpolysiloxane, viscosity at 25°C: 30,000,000 $mm^2/s$ (degree of polymerization: 3,747)
*6: X-21-5686, manufactured by Shin-Etsu Chemical Co., Ltd., dimethylpolysiloxane, viscosity at 25°C: 3,000,000 $mm^2/s$ (degree of polymerization: 2,680)
*7: KF-96H-500000cs, manufactured by Shin-Etsu Chemical Co., Ltd., dimethylpolysiloxane, viscosity at 25°C: 500,000 $mm^2/s$ (degree of polymerization: 1,972)
*8: KF-96H-300000cs, manufactured by Shin-Etsu Chemical Co., Ltd., dimethylpolysiloxane, viscosity at 25°C: 300,000 $mm^2/s$ (degree of polymerization: 1,790)
*9: KF-96H-100000cs, manufactured by Shin-Etsu Chemical Co., Ltd., dimethylpolysiloxane, viscosity at 25°C: 100,000 $mm^2/s$ (degree of polymerization: 1,429)
*10: KF-96H-10000cs, manufactured by Shin-Etsu Chemical Co., Ltd., dimethylpolysiloxane, viscosity at 25°C: 10,000 $mm^2/s$ (degree of polymerization: 804)
*11: KF-96H-1000cs, manufactured by Shin-Etsu Chemical Co., Ltd., dimethylpolysiloxane, viscosity at 25°C: 1,000 $mm^2/s$ (degree of polymerization: 357)
*12: KP-550, manufactured by Shin-Etsu Chemical Co., Ltd., isododecane solution of (acrylates/dimethicone) copolymer (40% by mass)

Regarding the degree of polymerization (P) of the above-mentioned components *2 and *5 to *11, the molecular weight (M) is obtained from the above-mentioned formula (4) from the viscosity ($\eta$), and the degree of polymerization (P) can be obtained from the following formula (5) since the molecular weight of the basic unit of dimethylpolysiloxane is 74.

$$P = M/74 \quad (5)$$

From Tables 1 to 4, according to the treatment method of the present invention, by performing one time treatment, color fading is reduced even after artificially colored hair is repeatedly washed, and color durability is good. In addition, it can be seen that an effect of preventing adhesion of dirt to hair can be imparted. In Comparative Example 1, a smooth film was not formed, and the evaluation of "Difficulty in Adhesion of Dirt" could not be performed.
In addition, from Table 5, it can be seen that according to the treatment method of the present invention, even in a case where regenerated collagen fibers which are fibers for head decoration products are used in place of hair, good color durability and an effect of preventing adhesion of dirt can be obtained.

Industrial Applicability

According to the present invention, it is possible to improve the color durability of a keratin substance or a fiber for a head decoration product which has been artificially colored or decolored even by one time treatment, and it is also possible to impart an effect of preventing adhesion of dirt.

## Claims

1. A method for treating an artificially colored or decolored keratin substance or fiber for a head decoration product with a composition, the method comprising a step of applying the composition to the keratin substance or the fiber for a head decoration product, wherein the composition comprises the following component (A) and component (B):

    (A) a silicone film-forming agent;
    (B) an organopolysiloxane having a degree of polymerization of 100 or more, and

    wherein a proportion of a content mass of the component (A) to a total content mass of the component (A) and the component (B) [(A)/{(A) + (B)}] is 10% or more.

2. The method according to claim 1, wherein the content of the component (A) in the composition is less than 25% by mass.

3. The method according to claim 1 or 2, wherein the component (B) has a viscosity at 25°C of 120 $mm^2/s$ or more

and 100,000,000 $mm^2/s$ or less.

4. The method according to any one of claims 1 to 3, wherein the component (A) is one or more selected from the group consisting of the following component (A1) and component (A2):

(A1): a silicone resin represented by an average formula $(R^1)_mSiO_{(4-m)/2}$ (wherein, $R^1$ represents a hydrocarbon group having 1 or more and 12 or less carbon atoms which may be fluorine-substituted or a hydroxy group; a plurality of $R^1$'s may be the same as or different from each other; and m is an average number which is more than 0 and less than 3), the silicone resin comprising one or more units selected from the group consisting of a T unit represented by $R^1SiO_{3/2}$ and a Q unit represented by $SiO_{4/2}$; and
(A2): a silicone polymer comprising a polysiloxane moiety and a moiety formed of a non-silicone organic chain.

5. The method according to any one of claims 1 to 4, wherein the composition is applied to a keratin substance or a fiber for a head decoration product in a dry state.

6. A kit for treating a keratin substance or a fiber for a head decoration product, comprising a first agent comprising one or more selected from the group consisting of a colorant and a decolorant, and a second agent comprising the following component (A) and component (B), wherein a ratio of a content mass of the component (A) to a total content mass of the component (A) and the component (B) [(A)/{(A) + (B)}] is 10% or more:

(A) a silicone film-forming agent;
(B) an organopolysiloxane having a degree of polymerization of 100 or more.

## INTERNATIONAL SEARCH REPORT

International application No.
**PCT/JP2022/043784**

**A. CLASSIFICATION OF SUBJECT MATTER**

***A61K 8/89***(2006.01)i; ***A61K 8/891***(2006.01)i; ***A61K 8/893***(2006.01)i; ***A61Q 3/02***(2006.01)i; ***A61Q 5/10***(2006.01)i
FI: A61K8/89; A61Q5/10; A61Q3/02; A61K8/891; A61K8/893

According to International Patent Classification (IPC) or to both national classification and IPC

**B. FIELDS SEARCHED**

Minimum documentation searched (classification system followed by classification symbols)

A61K8/89; A61K8/891; A61K8/893; A61Q3/02; A61Q5/10

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | US 2009/0074702 A1 (L'OREAL S.A.) 19 March 2009 (2009-03-19) claims, paragraphs [0001], [0008]-[0010], [0141]-[0168], [0354], [0358], [0359], [0363] | 1–6 |
| X | JP 2013-541585 A (AVON PRODUCTS, INC.) 14 November 2013 (2013-11-14) claims, paragraphs [0006], [0007], [0009], [0036], [0046], [0076], [0078], [0103], [0104], [0107], [0108] | 1-6 |
| A | JP 2011-519853 A (AVON PRODUCTS, INC.) 14 July 2011 (2011-07-14) entire text | 1-6 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 January 2023** | **31 January 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/043784**

| Patent document cited in search report | Publication date (day/month/year) | Patent family member(s) | Publication date (day/month/year) |
|---|---|---|---|
| US 2009/0074702 A1 | 19 March 2009 | (Family: none) | |
| JP 2013-541585 A | 14 November 2013 | US 2012/0110752 A1<br>claims, paragraphs [0005], [0006], [0008], [0035], [0045], [0078], [0080], [0105], [0106], [0109]<br>WO 2012/061025 A1<br>EP 2635349 A1 | |
| JP 2011-519853 A | 14 July 2011 | US 2009/0274640 A1<br>entire text<br>WO 2009/134555 A2<br>EP 3023091 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2013541585 A
- JP 2003342139 A
- JP H111530 A
- JP 2000063225 A
- JP H225411 A
- JP H2132141 A
- JP H3162442 A
- JP 2003104825 A
- JP H692825 A